(19) Europäisches Patentamt / European Patent Office / Office européen des brevets

(11) **EP 4 623 908 A1**

(12) **EUROPEAN PATENT APPLICATION**
published in accordance with Art. 153(4) EPC


(43) Date of publication:
**01.10.2025 Bulletin 2025/40**

(21) Application number: **23893742.9**

(22) Date of filing: **17.11.2023**

(51) International Patent Classification (IPC):
***A61K 31/353** (2006.01)* ***A61K 9/72** (2006.01)*
***A61K 9/14** (2006.01)* ***A61P 11/00** (2006.01)*

(52) Cooperative Patent Classification (CPC):
**A61K 9/0073; A61K 9/14; A61K 31/353; A61P 11/00**

(86) International application number:
**PCT/CN2023/132299**

(87) International publication number:
**WO 2024/109652 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.11.2022 CN 202211485124**



(71) Applicants:
- **CF Pharmtech, Inc.**
  **Jiangsu 215143 (CN)**
- **Ruijin Hospital, Shanghai Jiaotong University School of Medicine**
  **Shanghai 200025 (CN)**
- **CF Pharmtech Guangzhou Limited**
  **Guangzhou, Guangdong 510700 (CN)**

(72) Inventors:
- **QU, Jieming**
  **Shanghai 200025 (CN)**
- **ZHAO, Jingya**
  **Shanghai 200025 (CN)**
- **XU, Lin**
  **Suzhou, Jiangsu 215143 (CN)**
- **CHE, Lin**
  **Suzhou, Jiangsu 215143 (CN)**
- **LI, Xiujuan**
  **Suzhou, Jiangsu 215143 (CN)**
- **QUAN, Mengxue**
  **Suzhou, Jiangsu 215143 (CN)**
- **LIANG, Bill Wenqing**
  **Suzhou, Jiangsu 215143 (CN)**

(74) Representative: **Metida**
**Gyneju str. 16**
**01109 Vilnius (LT)**


(54) **USE OF (-)-EPIGALLOCATECHIN GALLATE COMPOUND**


(57) The present invention relates to the field of chemical pharmaceuticals and provides use of an (-)-epigallocatechin gallate (EGCG) compound. The present invention particularly relates to use of an EGCG compound in the preparation of an inhalation drug for preventing and/or treating pulmonary fibrosis diseases, an inhalable pharmaceutical composition for preventing and/or treating pulmonary fibrosis diseases, and a method for preventing and/or treating pulmonary fibrosis diseases. In the use, the EGCG compound is EGCG or a pharmaceutically acceptable salt thereof, an ester thereof, a hydrate thereof, or a solvate thereof. The drug/pharmaceutical composition taking the EGCG compound as an active ingredient provided by the embodiment is used as an inhalable pharmaceutical composition for preventing and treating pulmonary fibrosis, such that an administration dosage can be greatly reduced, a relatively wide treatment window is provided, and a relatively slight adverse reaction and a relatively low administration frequency are achieved, thereby providing a new treatment choice for a pulmonary fibrosis patient.


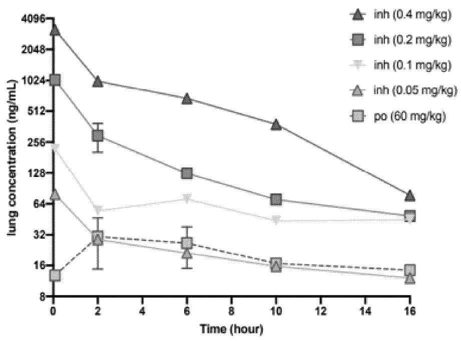


Fig. 4

EP 4 623 908 A1

## Description

### Priority

This PCT patent application claims priority to Chinese patent application No. 202211485124.4, filed on November 24, 2022, and incorporates the technical solutions of the aforementioned patent.

### Background of the Invention

Field of the Invention

The present disclosure relates to the field of pharmaceutical chemistry, and more particularly to the use of (-)-epigallocatechin gallate compound.

Description of Related Art

Pulmonary fibrosis (PF) is the terminal clinical manifestation of various interstitial lung diseases with different etiologies. It is characterized by persistent alveolar injury, fibroblast proliferation, and excessive extracellular matrix (ECM) deposition, leading to inflammation and fibrosis of the alveoli and lung interstitium, ultimately resulting in lung structural damage and respiratory failure. Therefore, PF is also referred to as interstitial lung disease (ILD) or diffuse parenchymal lung disease (DPLD). ILD is primarily classified into four major categories: idiopathic interstitial pneumonia (IIPs), interstitial lung fibrosis caused by autoimmune or connective tissue diseases, interstitial lung fibrosis induced by environmental exposure or medical treatments, and sarcoidosis. Among them, idiopathic pulmonary fibrosis (IPF) is the most significant and common type of IIPs. Current clinical research on pulmonary fibrosis primarily focuses on IPF, which manifests as progressive dyspnea accompanied by a persistent dry cough. Patients may also experience weight loss, fatigue, general discomfort, and musculoskeletal pain. The median survival time for IPF is approximately 2.8 years, with a 5-year survival rate of less than 50%, and most patients succumb to respiratory failure or secondary lung infections.

The pathogenesis of pulmonary fibrosis involves multiple factors, such as persistent alveolar epithelial injury leading to epithelial-mesenchymal transition (EMT), dysregulation of intracellular homeostasis, activation of signaling pathways including transforming growth factor-β (TGF-β), Wnt, and Notch, as well as epithelial cell dysfunction, apoptosis, and excessive scar tissue formation, all of which contribute to the disruption of pulmonary homeostasis. Currently, the only approved drugs for pulmonary fibrosis treatment worldwide are nintedanib and pirfenidone, both of which are administered orally. While these drugs can slow down the decline in lung function, they cannot reverse disease progression, and both are associated with high oral doses and severe adverse effects. Specifically, nintedanib is administered at 150 mg twice daily (BID), with the most common side effect being gastrointestinal reactions, diarrhea occurring in up to 61.5% of clinical study participants. Pirfenidone is taken at 801 mg three times daily (TID), with photosensitivity reactions observed in up to 51% of patients. These adverse effects often lead to dose reductions or treatment discontinuation.

Among the various cytokines that promote pulmonary fibrosis, TGF-β1 signaling is a key driver of collagen accumulation and fibrosis, as well as a crucial regulator of inflammation and epithelial cell proliferation. However, due to its multifunctional nature, the development of TGF-β1 inhibitors as therapeutic agents is significantly limited. Studies have shown that lysyl oxidase-like 2 protein (LOXL2) is an extracellular matrix protein that is rarely expressed in healthy adult tissues but can be induced in multiple fibrotic diseases and tumors. It is secreted by activated fibroblasts, disease-associated smooth muscle cells, endothelial cells, and epithelial cells. The combined inhibition of LOXL2 and TGF-β1 activity by trihydroxyphenolics compounds has been demonstrated to effectively block pathological collagen accumulation in vivo without causing the toxicity associated with global inhibitors.

(-)-Epigallocatechin gallate (EGCG) is the most abundant active component in green tea extract (GTE) catechins, accounting for 50-80% of the total catechin content. It is a polyphenolic flavonoid compound, and its chemical structure is denoted as

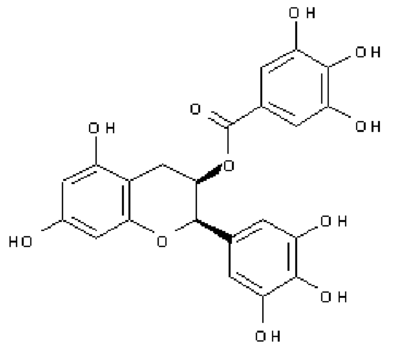

EGCG has various biological functions, including preventing cell damage caused by free radicals, exhibiting antibacterial properties, reducing inflammation, and preventing certain chronic diseases, such as heart disease, diabetes, and some cancers. Studies have shown that EGCG can effectively inhibit lysyl oxidase-like protein-2 (LOXL2) and TGFβ receptor 1 and 2 (TGFβR1/2) kinases (Ying Wei, et al. 2017; Harold A. Chapman, et al. 2020). **In** current research on EGCG related to pulmonary fibrosis, EGCG is administered orally. Although oral administration of EGCG has shown some effectiveness in pulmonary fibrosis animal models and human patients, its absorption in the gastrointestinal tract is poor, and drug absorption is affected by food. EGCG is hydrolyzed by esterases in the saliva after oral administration, and upon entering the bloodstream and liver, it undergoes extensive enzymatic metabolism, such as glucuronidation and sulfation, resulting in very low bioavailability. A high dosage is required to potentially exert anti-pulmonary fibrosis effects. For example, a dose of 600 mg is reported in the literature by Harold A. Chapman, et al. Long-term high-dose administration may also pose safety risks.

Additionally, there is evidence from published human or animal studies that links oral Green Tea Extract (GTE) to liver damage (Jiang Hu, et al., 2018; García-Cortés et al., 2016; Harrison-Dunn, 2016; Teschke et al., 2014). In the safety assessment of EGCG, published by the European Food Safety Authority (EFSA) Scientific Cooperation Project (ESCO) in 2018, intervention clinical trial evidence showed that daily oral intake of EGCG at doses equal to or higher than 800 mg significantly increased serum transaminases in subjects, indicating liver damage, and should not be considered safe. It has also been reported that a product containing 80% GTE with a daily dose equivalent to 375 mg of EGCG also exhibited hepatotoxicity (EFSA, 2018). Furthermore, clinical studies have shown that oral EGCG can significantly reduce the body weight of patients (I-Ju Chen, et al., 2016). For IPF patients, those with lower body weight have higher mortality and shorter survival than patients with higher body weight. The median survival for patients with BMI $<$ 25 and BMI $\geq$ 30 is approximately 3.6 years and 5.8 years, respectively (Mazen Alakhras, et al., 2007; Nobuyasu Awano, et al., 2021). Therefore, the safe and effective window for oral EGCG treatment of IPF is narrow, with the risk of ineffectiveness at low doses and toxicity at high doses. Due to the complexity of the disease, IPF patients often require dose adjustments according to disease progression, and oral EGCG cannot meet the individualized treatment needs of patients. After oral administration, EGCG undergoes extensive enzymatic metabolism in the body. Although EGCG is not a substrate of the CYP450 enzyme system, it can inhibit the activity of several CYP enzymes to varying degrees. EGCG can also inhibit drug transporters such as OATP. Therefore, EGCG may have significant effects on the bioavailability of various drugs. Some researchers suggest that patients receiving treatment with OATP substrate drugs, especially those with a narrow therapeutic index, should avoid or at least cautiously consume large amounts of GTE or EGCG (Ahmed A. Albassam, 2017). In a study of 26 IPF patients, co-administration of EGCG significantly reduced the plasma exposure of Nintedanib by about 21%, which is a statistically significant effect and may impair efficacy (G. D. Marijn Veerman, et al., 2022). Thus, due to the narrow therapeutic window and severe drug-drug interactions, the clinical application of oral EGCG in treating pulmonary fibrosis is limited.

Currently, there are no inhalation drugs successfully approved for the treatment of IPF globally, and the development of related inhalation drugs is also rare. Pirfenidone requires a very large oral dose to achieve an effective drug level in the lungs, with the approved dose being 801 mg TID. This results in very high plasma drug levels, leading to poor patient tolerance. Its nebulized inhalation formulation is under development, but in Phase II trials, it was discontinued for low-dose (50 mg/once per day) inefficacy, and the ongoing high-dose group (100 mg/ twice a day) has higher inhalation doses and more frequent administration. Current clinical performance has not significantly outperformed oral administration as initially expected. Animal experiments show that after changing the administration of pirfenidone to inhalation, the drug's half-life in the lungs is only about 10 minutes. In Phase I studies, human pharmacokinetics (PK) were basically consistent with animal results. A nebulized dose of 100 mg pirfenidone only raised the maximum concentration (Cmax) in epithelial lining fluid (ELF) in the lungs 35 times higher than the Cmax of the oral dose (801 mg), and it rapidly declined to a lower lung concentration than oral administration. The systemic exposure was about 15 times lower than the oral dose, and pirfenidone inhalation still resulted in higher plasma drug concentrations (807-1370 ng/mL), thus demonstrating that pirfenidone, after inhalation administration, still presents a high risk of adverse reactions. This indicates that pirfenidone

rapidly enters the bloodstream from the lungs after inhalation and is unable to maintain effective drug concentrations in the lungs for extended periods, failing to achieve the expected ideal effect.

It is evident that not all drugs are suitable for pulmonary delivery, as most drugs rapidly enter the bloodstream after inhalation due to their inability to maintain effective lung retention, making it impossible to maintain high concentrations in the lungs and achieve sustained therapeutic effects. Additionally, due to the complex pathogenesis of pulmonary fibrosis and the rapid progression, drug development is extremely challenging, with a high failure rate. Various growth factors involved in fibrosis, such as TGF-β and platelet-derived growth factor (PDGF), have multiple physiological functions in the body, maintaining homeostasis, and systemic inhibition of these growth factors often leads to toxic side effects. Therefore, therapeutic drugs for pulmonary fibrosis should have a broad therapeutic window, provide individualized treatment plans for patients with different severities, and ideally be deliverable through inhalation to exert long-lasting effects in the lungs. This remains an unmet clinical need.

Hence, the current technology still requires improvement and development.

## Summary of the Invention

In view of the deficiencies of the existing technology, the purpose of this disclosure is to provide an application of (-)-Epigallocatechin gallate (EGCG) and its derivatives, aiming to solve the issues with the oral administration of EGCG in the treatment of pulmonary fibrosis, such as extremely low bioavailability, the need for high doses, a narrow therapeutic window, and severe drug-drug interactions.

The technical solution of this disclosure is as follows:
In the first aspect, this disclosure provides an inhalable pharmaceutical composition comprising (-)-epigallocatechin gallate compound for use in prevention and/or treatment of pulmonary fibrosis, wherein the (-)-Epigallocatechin gallate compound is (-)-Epigallocatechin gallate or its pharmaceutically acceptable salts, esters, hydrates, or solvates.

In the second aspect, this disclosure provides an inhalable pharmaceutical composition for the prevention and/or treatment of pulmonary fibrosis, which includes: (-)-Epigallocatechin gallate or its derivative as the active ingredient, and pharmaceutically acceptable excipients; wherein the (-)-Epigallocatechin gallate compound is EGCG ((-)-Epigallocatechin gallate) or its pharmaceutically acceptable salts, esters, hydrates, or solvates.

In the third aspect, this disclosure provides a method for the prevention and/or treatment of pulmonary fibrosis, wherein the method includes: administering the (-)-Epigallocatechin gallate compound to a subject through inhalation, where the (-)-Epigallocatechin gallate compound is (-)-Epigallocatechin gallate or its pharmaceutically acceptable salts, esters, hydrates, or solvates.

Beneficial effects:

Compared to the existing oral administration of EGCG for the treatment of pulmonary fibrosis, the present disclosure involves the use of EGCG or its pharmaceutically acceptable salts, esters, hydrates, or solvates in the preparation of inhalation drugs for the treatment of pulmonary fibrosis, thereby achieving:

1. Reduced dosage and dosing frequency;
2. Reduced adverse reactions and side effects;
3. Increased drug concentration and retention time in the lungs;
4. Broader safe and effective therapeutic window;
5. Minimal impact on the bioavailability of other drugs used in combination and no drug-drug interactions with other medications.

## Brief Description of the Figures

Figure 1: A comparison of plasma and lung drug concentrations (ng/mL) over time (hour) in rats after tracheal nebulization of EGCG at different doses (inh, doses of 1.6 mg/kg, 3.2 mg/kg, 6.4 mg/kg).

Figure 2: A comparison of plasma and lung drug concentrations (ng/mL) over time (hour) in rats after intravenous administration (iv, dose of 3.2 mg/kg) and oral gavage (po, dose of 60 mg/kg) of EGCG.

Figure 3: A comparison of plasma and lung drug concentrations (ng/mL) over time (hour) in rats after tracheal nebulization of EGCG solution (dose of 0.8 mg/kg) and oral gavage of EGCG solution (dose of 60 mg/kg).

Figure 4: A comparison of lung drug concentrations (ng/mL) over time (hour) in rats after tracheal nebulization of low-dose EGCG (doses of 0.05 mg/kg, 0.1 mg/kg, 0.2 mg/kg, 0.4 mg/kg) and oral gavage of EGCG solution (dose of 60 mg/kg).

Figure 5: A comparison of the lung weight index (%) of mice after administration of EGCG solution via tracheal nebulization and oral gavage compared to the pulmonary fibrosis model group (Kruskal-Wallis test, *: $p<0.05$, statistically significant difference; **: $p<0.01$, highly significant difference; ***: $p<0.001$, extremely significant difference).

Figure 6: A comparison of the body weight (g) of mice after administration of EGCG via tracheal nebulization and oral gavage versus the pulmonary fibrosis model group (statistical analysis performed using two-way ANOVA and Tukey's multiple comparison test).

Figure 7a: The effect of EGCG administered via tracheal nebulization and oral gavage on the degree of lung fibrosis in mice induced by bleomycin (Masson stain, 100×).

Figure 7b: The effect of EGCG administered via tracheal nebulization and oral gavage on the histopathological changes in the lung tissue of mice induced by bleomycin (H&E stain, 100×).

Figure 8: The effect of EGCG solution administered via tracheal nebulization compared to oral gavage on alveolar inflammation cell infiltration, hemorrhage, alveolar expansion, and alveolar fibrous exudates in pulmonary fibrosis mice.

Figure 9: A comparison of the fibrosis score (Fibrosis Score) of mice after EGCG administration via tracheal nebulization and oral gavage versus the pulmonary fibrosis model group.

Figure 10: A comparison of the hydroxyproline content (μg/μL) in lung tissue of mice after EGCG administration via oral gavage and tracheal nebulization versus the pulmonary fibrosis model group (One-Way ANOVA and Dunnett's test for inter-group comparison).

Figure 11: A comparison of the hydroxyproline content (μg/μL) in lung tissue of rats after EGCG administration via oral gavage and tracheal nebulization , and after administration of the control drug pirfenidone (AP01) via tracheal nebulization, versus the pulmonary fibrosis model group (Kruskal-Wallis test, *: $p<0.05$, statistically significant difference; **: $p<0.01$, highly significant difference; ***: $p<0.001$, extremely significant difference; : $p<0.0001$, extremely highly significant difference).

Figure 12: A comparison of the average plasma concentration of EGCG over time in healthy subjects after oral administration of EGCG capsules (150mg/capsule, 4 capsules total) and nebulized EGCG solution (3 mg, 10 mg, 30 mg dose group).

## Detailed Description of the Invention

This disclosure provides the use of (-)-epigallocatechin gallate compound. To clarify the purpose, technical solution, and effects of this disclosure, the following detailed description is provided. It should be understood that the specific embodiments described here are for explanatory purposes and are not intended to limit the scope of this disclosure.

In the first aspect, the embodiments of the present disclosure provide an inhalable pharmaceutical composition comprising (-)-epigallocatechin gallate compound or its pharmaceutically acceptable salts, esters, hydrates, or solvates for use in prevention and/or treatment of pulmonary fibrosis.

In some embodiments, the (-)-epigallocatechin gallate compound is (-)-epigallocatechin gallate or a pharmaceutically acceptable ester thereof. In certain embodiments, the pharmaceutically acceptable ester refers to a fatty acid ester of EGCG, wherein at least one hydroxyl group of the EGCG structure is esterified with a C1-C30 fatty acid.

In some embodiments, the inhalable pharmaceutical composition is formulated into a dosage form suitable for inhalation, selected from solutions, suspensions, aerosols (such as nebulized inhalations), or dry powder inhalations.

In some embodiments, the inhalable pharmaceutical composition is prepared by processing the (-)-epigallocatechin gallate compound into a powder through a drying process, which is then reconstituted using a diluent.

In some embodiments, when the dosage form is a solution, the inhalable pharmaceutical composition includes: the (-)-epigallocatechin gallate compound as the active ingredient, diluent(s), and at least one or more pH modifiers, osmotic pressure regulators, and antioxidants, with the (-)-epigallocatechin gallate compound completely dissolved in the diluent.

In some embodiments, when the dosage form is a suspension, the inhalable pharmaceutical composition includes: the (-)-epigallocatechin gallate compound as the active ingredient, diluent(s), and one or more selected from surfactants, pH modifiers, and tension regulators. The (-)-epigallocatechin gallate compound combines with pharmaceutically acceptable carriers for inhalation administration, forming particles that are suspended in a diluent.

In some embodiments, when the dosage form is an aerosol, the inhalable pharmaceutical composition includes: the (-)-epigallocatechin gallate compound as the active ingredient, diluent(s), propellant(s), and one or more selected from surfactants, solubilizers, and pH modifiers.

In some embodiments, when the dosage form is a dry powder inhalation, the inhalable pharmaceutical composition includes: the (-)-epigallocatechin gallate compound as the active ingredient, pharmaceutically acceptable carrier(s) for inhalation, and one or more selected from fillers and surfactants.

In some embodiments, the diluent is one or more of water, ethanol, and glycerol; preferably, the diluent is water.

In some embodiments, the pH of the inhalable pharmaceutical composition ranges from 3.0 to 5.0.

In some embodiments, the concentration of (-)-epigallocatechin gallate in the inhalable pharmaceutical composition ranges from 0.1 mg/mL to 25 mg/mL.

In some embodiments, the inhalable pharmaceutical composition includes only the (-)-epigallocatechin gallate compound, or it includes both (-)-epigallocatechin gallate and other anti-pulmonary fibrosis drugs.

In some embodiments, the other anti-pulmonary fibrosis drugs are selected from pirfenidone, nintedanib, corticosteroids, immunosuppressants, prostacyclin and its analogs, CTGF antibodies, galectin-3 inhibitors, integrin antagonists, recombinant serum amyloid P and its analogs, PDE inhibitors, LPA antagonists, JAK kinase inhibitors, or one or more cytokine receptor TKIs.

Preferably, in some embodiments, the other anti-pulmonary fibrosis drugs are selected from pirfenidone, nintedanib, BI 1015550 ([1-({(5R)-2-[4-(5-chloropyrimidin-2-yl)piperidin-1-yl]-5-oxido-6,7-dihydrothieno[3,2-d]pyrimidin-4-yl}amino)cyclobutyl]methanol, CAS No.: 1423719-30-5,

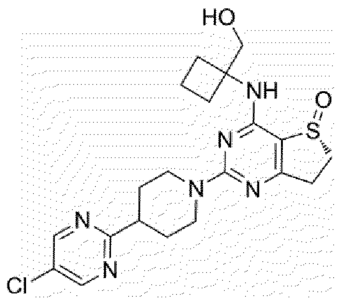

), treprostinil and its analogs, recombinant serum amyloid P, LPA antagonists, or one or more of these.

In some embodiments, the inhalation drug may also be combined with the other anti-fibrosis drugs described above. The term "combination therapy (i.e. be combined with the other drugs)" as used in the present invention refers to a method of administration in which at least one dose of (-)-epigallocatechin gallate (EGCG) or its pharmaceutical salt, ester, hydrate, or solvated form, is administered in combination with at least one dose of another compound, both of which exhibit pharmacological effects. The time period for administration is a dosing cycle, preferably within 24 hours, more preferably within 12 hours. The (-)-epigallocatechin gallate and the other anti-fibrosis drug can be administered either simultaneously or sequentially. This period includes treatments where (-)-epigallocatechin gallate and the other anti-fibrosis drug are administered via the same or different routes of administration. The combined administration method of the present invention can be selected from simultaneous administration, independent preparation and co-administration, or independent preparation and successive administration.

In some embodiments, the administered dose of (-)-epigallocatechin gallate ranges from 0.1 mg to 100 mg per dose.

Preferably, in some embodiments, the administered dose of (-)-epigallocatechin gallate ranges from 0.1 mg to 50 mg per dose.

More preferably, in some embodiments, the administered dose of (-)-epigallocatechin gallate ranges from 0.1 mg to 30 mg per dose.

Most preferably, in some embodiments, the administered dose of (-)-epigallocatechin gallate ranges from 0.1 mg to 15 mg per dose.

Exemplarily, the administered dose of (-)-epigallocatechin gallate may be selected from 0.1 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 25 mg, 30 mg, 35 mg, 40 mg, 45 mg, 50 mg, 55 mg, 60 mg, 65 mg, 70 mg, 75 mg, 80 mg, 90 mg, 100 mg per dose, etc.

The oral dose of EGCG reported in the literature is 400 mg to 600 mg per dose. In some embodiments, the administered dose of (-)-epigallocatechin gallate ranges from 1/1000 to 1/10 of the oral dose.

Preferably, in some embodiments, the administered dose of (-)-epigallocatechin gallate is 1/600 to 1/10 of the oral dose. More preferably, in some embodiments, the dose of (-)-epigallocatechin gallate ranges from 1/200 to 1/10 of the oral dose.

In some embodiments, the pulmonary fibrosis is interstitial lung disease, including idiopathic pulmonary fibrosis, lung interstitial fibrosis caused by autoimmune or connective tissue diseases, occupational or exposure-related interstitial lung fibrosis, treatment-induced interstitial lung fibrosis, and one or more forms of sarcoidosis.

In some embodiments, idiopathic interstitial pneumonia includes idiopathic pulmonary fibrosis, lung interstitial

fibrosis caused by autoimmune or connective tissue diseases includes lupus, scleroderma, polymyositis or dermatomyositis, and rheumatoid arthritis-associated interstitial lung diseases, exposure-related interstitial fibrosis includes asbestosis, silicosis, and hypersensitivity pneumonitis; treatment-induced interstitial fibrosis includes interstitial lung disease caused by chemotherapy, radiotherapy, and certain drug treatments.

In the second aspect, the embodiments of the present disclosure provide an inhalable pharmaceutical composition for the prevention and/or treatment of pulmonary fibrosis, comprising: (-)-epigallocatechin gallate (EGCG) or a pharmaceutically acceptable salt, ester, hydrate, or solvate thereof as the active ingredient, and pharmaceutically acceptable excipients.

In some embodiments, the content of the (-)-epigallocatechin gallate in the inhalable pharmaceutical composition ranges from 0.1 mg to 100 mg, 0.1 mg to 80 mg, 0.1 mg to 70 mg, 0.1 mg to 60 mg, 0.1 mg to 50 mg, 0.1 mg to 30 mg, 0.1 mg to 15 mg, etc.

In some embodiments, the content of the (-)-epigallocatechin gallate in each dose of the inhalable pharmaceutical composition ranges from 0.1 mg to 50 mg.

In some embodiments, the content of the (-)-epigallocatechin gallate in each dose of the inhalable pharmaceutical composition ranges from 0.5 mg to 30 mg.

In some embodiments, the content of the (-)-epigallocatechin gallate in each dose of the inhalable pharmaceutical composition is, for example, 0.5 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 25 mg, 30 mg.

In one embodiment, the inhalable pharmaceutical composition is prepared by combining the (-)-epigallocatechin gallate with pharmaceutically acceptable excipients using conventional or specialized formulation techniques to produce an inhalable dosage form, which may be an inhalation solution, inhalation suspension, aerosol, dry powder inhalation, or other inhalable formulations.

Furthermore, in some embodiments, the inhalation solution is a nebulized inhalation solution, and the inhalation suspension is a nebulized inhalation suspension. Nebulized inhalation refers to the process of using a nebulizer device to disperse the drug composition into fine mist droplets, suspending them in a gas, which can then be inhaled into the respiratory tract and lungs. Nebulized inhalation allows for local treatment and systemic effects, such as strong anti-fibrotic action and anti-inflammatory effects.

In some embodiments, the (-)-epigallocatechin gallate is first processed into a powder by a drying process, and before administration to the subject, the powder is reconstituted with a diluent and then delivered in a nebulized form to the lungs of the subject. The drying process may include freeze-drying, spray-drying, spray-freeze drying, or supercritical fluid technology.

In some embodiments, the dosage form of the inhalable pharmaceutical composition is an inhalation solution, where the pharmaceutically acceptable excipients include surfactants, pH modifiers, antioxidants, preservatives, osmotic pressure regulators, metal ion chelating agents, water, and additives, in one or more combinations. In other embodiments, the dosage form of the inhalable pharmaceutical composition is an inhalation suspension, where the pharmaceutically acceptable excipients also include surfactants, pH modifiers, antioxidants, preservatives, osmotic pressure regulators, metal ion chelating agents, water, and additives, in one or more combinations. Furthermore, in some embodiments, the inhalation solution or inhalation suspension contains little to no preservatives.

In some embodiments, the concentration of (-)-epigallocatechin gallate in the inhalation solution or inhalation suspension ranges from 0.1 mg/mL to 35 mg/mL, 0.1 mg/mL to 30 mg/mL, 0.1 mg/mL to 25 mg/mL, 0.5 mg/mL to 25 mg/mL, 0.5 mg/mL to 15 mg/mL, 0.5 mg/mL to 10 mg/mL, 0.5 mg/mL to 5 mg/mL, etc.

In some embodiments, the concentration of (-)-epigallocatechin gallate in the inhalation solution and inhalation suspension is, for example, 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL.

In some embodiments, the dosage form of the inhalable pharmaceutical composition is an aerosol, and the pharmaceutically acceptable excipients are selected from solvents, surfactants, propellants, and additives in one or more combinations.

Furthermore, in some embodiments, the propellant is a hydrofluoroalkane compound. Preferably, the propellant is one or both of 1,1,1,2-tetrafluoroethane (HFA134a) and 1,1,1,2,3,3,3-heptafluoropropane (HFA227). Further, in some embodiments, the additive includes a solvent, which is selected from glycerin, propylene glycol, polyethylene glycol, ethanol, or oleic acid, in one or more combinations. Preferably, the solvent is one or both of ethanol and propylene glycol.

In some embodiments, the dosage form of the inhalable pharmaceutical composition is a dry powder inhalation, and the pharmaceutically acceptable excipients include excipients, carriers, and additives. Further, in some embodiments, the excipients include carbohydrates, sugar alcohols, starches, macromolecular polymers, fatty acids or their salts, waxes, calcium sulfate, calcium carbonate, talc, iron oxide, and light anhydrous silicates, in one or more combinations. The carbohydrates include one or more of lactose, glucose, sucrose, trehalose, and others; the sugar alcohols include one or

more of erythritol, mannitol, sorbitol; the macromolecular polymers include one or more of crystalline cellulose, methyl cellulose, hydroxypropyl cellulose, carboxymethyl cellulose calcium, hydroxypropyl methyl cellulose, sodium carboxymethyl cellulose, branched starch, dextrin, arabic gum, agar, gelatin, astragalus gum, sodium alginate, polyvinylpyrrolidone, polyvinyl alcohol, and others; the fatty acids may include one or more of stearic acid, oleic acid, and others.

Preferably, in some embodiments, the excipients are selected from carbohydrates, sugar alcohols, macromolecular polymers, and calcium carbonate in one or more combinations. The carbohydrates are lactose or sucrose; the sugar alcohols are erythritol, mannitol, or sorbitol; the macromolecular polymers are carboxymethyl cellulose calcium, branched starch, polyvinylpyrrolidone, or methyl cellulose. Most preferably, the excipients are lactose or erythritol.

In some embodiments, the carrier includes one or more of lactose, glucose, fructose, sucrose, maltose, dextran, erythritol, sorbitol, mannitol, calcium sulfate, calcium carbonate, talc, or iron oxide.

In some embodiments, the form of the catechins is crystalline or amorphous.

In some embodiments, for the prevention and/or treatment of pulmonary fibrosis, the inhalable pharmaceutical composition is delivered to the subject's respiratory tract and lungs via an inhalation delivery device that emits aerosol droplets or micro-powdered particles. Further, in some embodiments, the average particle size of the aerosol droplets ranges from 0.5 μm to 10 μm; for example, the average particle size of the aerosol droplets can range from 1μm to 8 μm, 1μm to 5 μm, 1μm to 3 μm, 2μm to 3 μm, etc. In further embodiments, the average particle size of the micropowdered particles is less than or equal to 20 μm; preferably less than or equal to 10 μm, more preferably 1μm to 9 μm, and most preferably 3μm to 8 μm. This particle size range allows the micro-powdered particles to reach the respiratory tract (e.g., bronchi) and lungs of the subject.

In the third aspect, the embodiments of the present disclosure provide a method for preventing and/or treating pulmonary fibrosis, wherein the method includes: administering a therapeutically effective amount of (-)-epigallocatechin gallate to subjects via inhalation, wherein the (-)-epigallocatechin gallate is (-)-epigallocatechin gallate or a pharmaceutically acceptable salt, ester, hydrate, or solvate thereof.

Specifically, in some embodiments, the method may include delivering an effective amount of (-)-epigallocatechin gallate to the lungs of a mammal by inhalation. Pulmonary fibrosis may include: idiopathic interstitial pneumonia, pulmonary interstitial fibrosis caused by autoimmune or connective tissue diseases, pulmonary interstitial fibrosis caused by exposure or treatment, and interstitial lung diseases such as sarcoidosis. Among these, idiopathic interstitial pneumonia includes idiopathic pulmonary fibrosis. Interstitial fibrosis caused by autoimmune or connective tissue diseases includes interstitial lung diseases associated with lupus, scleroderma, polymyositis or dermatomyositis, and rheumatoid arthritis. Interstitial fibrosis related to exposure or occupational exposure includes asbestosis, silicosis, and hypersensitivity pneumonitis. Treatment-related interstitial fibrosis includes interstitial lung diseases caused by chemotherapy, radiotherapy, and certain medications.

In some embodiments, the administered dose of (-)-epigallocatechin gallate ranges from 0.01 mg/kg to 2.0 mg/kg. Specifically, the administered dose of the active ingredient EGCG in the tested animals may range from 0.01 mg/kg to 2.0 mg/kg, which translates to a clinical administered dose for human patients of 0.1mg to 100 mg. The corresponding administered dose can be calculated based on clinical references or literature methods (E Boger, et al. 2016; Ramon Hendrickx, et al. 2018; Therese Ericsson, et al. 2017). Taking into account the patient's weight and other physiological conditions, in some embodiments, the human administered dose may range from 0.1 mg to 80 mg, 0.1 mg to 70 mg, 0.1 mg to 60 mg, 0.1 mg to 50 mg, 0.1 mg to 30 mg, 0.1 mg to 15 mg, and so on. In some embodiments, the human administered dose may be, for example, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 25 mg, 30 mg, and so on.

The oral administered dose of EGCG reported in existing literature ranges from 400 mg to 600 mg per dose. In some embodiments, the method includes delivering (-)-epigallocatechin gallate to the patient's lungs at a dose of 1/1000 to 1/10 of the oral dose. Preferably, it may range from 1/600 to 1/10 of the oral dose. For example, it may range from 1/200 to 1/10 of the oral dose, or 1/50 to 1/10 of the oral dose.

In some embodiments, the administered dose of (-)-epigallocatechin gallate may range from 0.1 mg to 80 mg per dose, 0.1 mg to 70 mg per dose, 0.1 mg to 50 mg per dose, 0.5 mg to 30 mg per dose, 0.5 mg to 15 mg per dose, and so on.

Most preferably, the administered dose of the (-)-epigallocatechin gallate compound ranges from 0.5 mg to 15 mg per dose.

Exemplary administered doses of the (-)-epigallocatechin gallate compound are 0.1 mg per dose, 1 mg per dose, 1.5 mg per dose, 2 mg per dose, 2.5 mg per dose, 3 mg per dose, 3.5 mg per dose, 4 mg per dose, 4.5 mg per dose, 5 mg per dose, 5.5 mg per dose, 6 mg per dose, 6.5 mg per dose, 7 mg per dose, 7.5 mg per dose, 8 mg per dose, 8.5 mg per dose, 9 mg per dose, 9.5 mg per dose, 10 mg per dose, 10.5 mg per dose, 11 mg per dose, 11.5 mg per dose, 12 mg per dose, 12.5 mg per dose, 13 mg per dose, 13.5 mg per dose, 14 mg per dose, 14.5 mg per dose, 15 mg per dose, 15.5 mg per dose, 16 mg per dose, 16.5 mg per dose, 17 mg per dose, 17.5 mg per dose, 18 mg per dose, 18.5 mg per dose, 19 mg per dose, 19.5 mg per dose, 20 mg per dose, 25 mg per dose, 30 mg per dose, 35 mg per dose, 40 mg per dose, 45 mg per dose, 50 mg per dose, 55 mg per dose, 60 mg per dose, 65 mg per dose, 70 mg per dose, 75 mg per dose, 80 mg per dose, 90 mg per dose,

100 mg per dose, etc.

Furthermore, in some embodiments, the treatment method includes delivering 0.1-100 mg of (-)-epigallocatechin gallate to the patient's lungs. Exemplary administered doses include 0.1 mg - 80 mg, 0.1 mg - 70 mg, 0.1 mg - 60 mg, 0.1 mg - 50 mg, or 0.1 mg - 30 mg of (-)-epigallocatechin gallate. Specifically, the lung delivery administered dose may be 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 25 mg, 30 mg, etc.

In some embodiments, the method includes administering the (-)-epigallocatechin gallate compound to the subject via inhalation, including: initially delivering a lower dose to the lungs of the subject and then progressively increasing the dose based on the subject's condition.

In some embodiments, the method further includes administering an active agent to the subject via inhalation, which may be administered simultaneously, separately, or sequentially with the (-)-epigallocatechin gallate compound.

In some embodiments, the active agent is selected from pirfenidone, nintedanib, glucocorticoids, immunosuppressants, prostacyclin and its analogs, CTGF antibodies, Galectin-3 inhibitors, integrin antagonists, recombinant pentraxin-2 (e.g., PRM-151) and its analogs, PDE inhibitors, LPA antagonists, JAK kinase inhibitors, or a combination of multiple cytokine receptor TKIs. Furthermore, in some embodiments, the prostacyclin and its analogs may optionally include treprostinil and iloprost; the CTGF antibody may optionally include Pamrevlumab; the Galectin-3 inhibitor may optionally include GB0139; the integrin antagonists include integrin ($\alpha v\beta 1$, $\alpha v\beta 6$) antagonists, and the integrin antagonists may optionally include PLN-74809; recombinant Pentraxin-2 may optionally include PRM-151; PDE inhibitors may optionally include BI 1015550; and LPA antagonists may optionally include BMS-986278.

In some preferred embodiments, the active agents are selected from pirfenidone, nintedanib, BI 1015550, treprostinil and its analogs, recombinant pentraxin-2, or LPA antagonists, or a combination of these.

In some embodiments, the administration frequency of the (-)-epigallocatechin gallate compound is once every 48 hours, once daily, or twice daily. Specifically, the compound may be administered to the subject once every 48 hours, once a daily, or twice a daily. Furthermore, in some embodiments, the daily dose of the compound administered to the subject is less than or equal to 150 mg; preferably, it is less than or equal to 100 mg. Exemplary daily doses include 150 mg, 140 mg, 120 mg, 100 mg, 80 mg, 70 mg, 60 mg, 50 mg, 40 mg, 35 mg, 30 mg, 25 mg, 20 mg, 19.5 mg, 19 mg, 18.5 mg, 18 mg, 17.5 mg, 17 mg, 16.5 mg, 16 mg, 15.5 mg, 15 mg, 14.5 mg, 14 mg, 13.5 mg, 13 mg, 12.5 mg, 12 mg, 11.5 mg, 11 mg, 10.5 mg, 10 mg, 9.5 mg, 9 mg, 8.5 mg, 8 mg, 7.5 mg, 7 mg, 6.5 mg, 6 mg, 5.5 mg, 5 mg, 4.5 mg, 4 mg, 3.5 mg, 3 mg, 2.5 mg, 1 mg, 0.5 mg, 0.2 mg, 0.1 mg, and so on.

In some embodiments, the inhalation administration is performed via an inhalation delivery device.

The inhalation device may include a nebulizer, pressurized metered-dose inhaler, powder inhaler, or soft mist inhaler.

It should be understood that the doses described in this disclosure can refer to the output dose from the inhalation device (i.e., the device-delivered dose), or they can be converted to the dose delivered to the subject's lungs based on the general understanding in the field that inhalation devices have a delivery efficiency of approximately 25%-75% (i.e., the lung dose).

For human subjects, the most preferred dose of (-)-epigallocatechin gallate per administration ranges from 1 mg to 15 mg per dose, and the inhaled dose administered to the subject per administration ranges from 3 mg to 50 mg per dose.

The pharmaceutical compositions containing (-)-epigallocatechin gallate as the active ingredient, provided by the disclosed embodiments, can be administered via inhalation, delivering the drug to the subject's lungs effectively. After administration, the (-)-epigallocatechin gallate compound can be effectively deposited in the subject's lungs, resulting in higher lung drug concentrations and lower systemic drug concentrations, thus avoiding potential liver toxicity and other side effects. This creates a larger therapeutic window between efficacy and toxicity, making it feasible to have a wider range of treatment options.

Furthermore, the higher lung concentration of (-)-epigallocatechin gallate provided by the inhalation method can more quickly and effectively regulate key proteins associated with pulmonary fibrosis and inflammation, such as $\alpha$-smooth muscle actin ($\alpha$-SMA), SNAI1, collagen **I,** fibronectin, phosphorylated SMAD3 (pSMAD3), and phosphorylated SMAD2 (pSMAD2), resulting in better efficacy. Some of these proteins cannot be effectively inhibited by oral administration of EGCG. Inhalation delivery of the drug composition allows (-)-epigallocatechin gallate to maintain a higher drug concentration in the subject's lungs for a longer time, which enhances patient compliance by reducing the frequency of administration. Additionally, inhalation avoids the potential drug-drug interactions that could occur between oral EGCG and other IPF treatments.

Overall, the pharmaceutical compositions containing (-)-epigallocatechin gallate as an effective ingredient, used as an inhalable pharmaceutical composition for the prevention and treatment of pulmonary fibrosis, can significantly reduce the required dosage, provide a broader therapeutic window, minimize adverse reactions, and reduce administration frequency. This offers a new treatment option for pulmonary fibrosis patients, with significant social and economic benefits.

The following provides a detailed explanation of the technical solution of this disclosure through specific examples.

Example 1: Plasma pharmacokinetics study in rats after administration of EGCG via tracheal nebulization, intravenous injection, and oral gavage.

1.1 Preparation

Materials: EGCG, content > 98wt%.

Preparation of tracheal nebulization solution: Accurately weigh the EGCG compound and dissolve it in Solvent 1 to prepare a clear and transparent drug solution. Solvent 1 is composed of purified water, citric acid, and sodium citrate.

Preparation of intravenous injection and oral gavage solutions: Accurately weigh the EGCG compound and dissolve it in saline.

Instruments: Liquid pulmonary drug delivery nebulizer.

Animals: Clean-grade male SD rats, provided by Qinglongshan Animal Breeding Farm in Jiangning District, Nanjing City, with a body weight range of 160-200g.

1.2 Administration Method and Dosage

Tracheal Nebulization (inh): Rats in each tracheal nebulization group were anesthetized with isoflurane and fixed in a restraint device. An anesthetic laryngoscope was used to press the animal's tongue root and expose the glottis. A micro liquid nebulizer needle (blunt tip) filled with a specific amount of EGCG solution, was gently inserted into the trachea. The drug was nebulized into the lungs, the needle was quickly withdrawn, and the rat was removed from the holder, with its head rotated left and right to ensure even distribution of the drug across the lung lobes. The nebulized inhalation doses were 1.6 mg/kg, 3.2 mg/kg, and 6.4 mg/kg, with 5 rats in each dose group.

Oral Gavage (po): The dosage for each rat was determined based on body weight. An appropriate size syringe and gavage tube were used to administer the drug orally. The dose was 60 mg/kg, with 5 rats.

Intravenous Injection (iv): EGCG was administered via tail vein injection. The dose was 3.2 mg/kg, with 5 rats in total.

1.3 Sample Collection and Processing Method

Blood samples were collected from the experimental groups via the orbital venous plexus before administration (0 min) and at 5 min, 15 min, 30 min, 1 h, 2 h, 4 h, and 6 h after administration (8 sampling points in total), with approximately 50-100 μL of whole blood collected at each point.

After collection at the specified time points, blood samples were placed in heparinized tubes, centrifuged, and plasma was separated into centrifuge tubes, then stored at -70°C for subsequent analysis.

1.4 Analytical Conditions and Pharmacokinetic Parameter Calculation Method

Mass spectrometry conditions: Determined using UPLC-MS/MS. Chromatographic conditions: Determined using. Pharmacokinetic parameters were calculated using the WinNonlin pharmacokinetic software with statistical moment methods for each animal.

1.5 Experimental Results and Data Analysis

After administration of EGCG via tracheal nebulization (inh, doses of 1.6 mg/kg, 3.2 mg/kg, 6.4 mg/kg), intravenous injection (iv, dose of 3.2 mg/kg), and oral gavage (po, dose of 60 mg/kg), the pharmacokinetic parameters in rat plasma are shown in Table 1. The time-concentration curves of EGCG in rat plasma and lungs after tracheal nebulization (inh, doses of 1.6 mg/kg, 3.2 mg/kg, 6.4 mg/kg) are compared in Figure 1. The time-concentration curves of EGCG in rat plasma and lungs after intravenous injection (iv, dose of 3.2 mg/kg) and oral gavage (po, dose of 60 mg/kg) are compared in Figure 2. The experimental results shown in Table 1 and Figures 1 and 2 indicate that after inhalation (such as tracheal nebulization), the bioavailability of EGCG is significantly increased. When achieving the same plasma exposure level, the dose ratio between oral gavage and inhalation is 37.5 times (po 60 mg/kg: inh 1.6 mg/kg), indicating that inhalation administration significantly reduces the dose of EGCG required.

Table 1: Plasma Pharmacokinetic Parameters in Rat After Administration of EGCG Via Tracheal Nebulization, Intravenous Injection, and Oral Gavage.

| Administration method | Dosage mg/kg | $C_{max}$ (ng/mL) | $AUC_{0-t}$ (h*ng/mL) | F (%) |
|---|---|---|---|---|
| tracheal nebulization (inh) | 1.6 | 298.4±91.9 | 409.3±75.6 | 48.7±9.0 |
| | 3.2 | 1119±127.1 | 1124±220.5 | 66.9±13.1 |
| | 6.4 | 2016±658.5 | 1858±594.6 | 55.3±17.7 |
| oral gavage (po) | 60 | 118.1±73.0 | 371.7±166.8 | 1.2±0.0 |
| intravenous injection (iv) | 3.2 | 1920±788.8 | 1681±614.4 | - |

Example 2: Study on the Changes in Drug Concentration in Rat Plasma and Lungs After Administration of EGCG via Tracheal Nebulization and Oral Gavage

As shown in Example 1, after inhalation (such as tracheal nebulization) administration of 1.6 mg/kg EGCG and oral gavage administration of 60 mg/kg EGCG, the AUC0-t in rat plasma were similar. Based on this, this example investigates the changes in drug concentration in rat plasma and lungs over time after a single lower dose of tracheal nebulization (0.8 mg/kg) compared to oral gavage (60 mg/kg) of EGCG. Both the tracheal nebulization and oral gavage groups consist of 5 rats each.

2.1 Blood Sampling Method: Blood was taken via femoral artery. Samples were taken at 30 minutes, 2 hours, 4 hours, and 6 hours post-administration (4 sampling points). After blood collection, 0.9 wt% physiological saline was used to lavage the lungs twice, then the entire lung was removed, dried with filter paper, and stored at -20°C until further analysis.

2.2 Plasma Sample Processing Method: 1 mL of plasma was mixed with 3 mL of ethyl acetate solution, vortexed, centrifuged, and the supernatant was evaporated to dryness. The residue was reconstituted with a reconstitution solution, vortexed, and 100 μL was used for analysis of plasma drug concentration.

2.3 Lung Tissue Sample Processing Method: Lung tissue samples were weighed, and 3 times the volume of physiological saline (w:v) was added to homogenize. 1 mL of the homogenate was mixed with 3 mL of ethyl acetate solution, vortexed, centrifuged, and the supernatant was evaporated to dryness. The residue was reconstituted with a reconstitution solution, vortexed, and 100 μL was used for analysis of lung drug concentration.

2.4 Experimental Results and Data Analysis:

After inhalation (tracheal nebulization) of 0.8 mg/kg EGCG solution and oral gavage of 60 mg/kg EGCG solution, the Cmax and AUC0-t of the drug in rat plasma and lungs are shown in Table 2. The time-concentration curves for plasma and lung drug concentrations after tracheal nebulization (0.8 mg/kg) and oral gavage (60 mg/kg) are compared in Figure 3. The results in Table 2 and Figure 3 show that after tracheal nebulization of 0.8 mg/kg EGCG and oral gavage of 60 mg/kg EGCG, the drug concentration levels in plasma and the time-concentration curves are similar. However, after inhalation, the drug concentration in the lungs was found to be 100 times higher than that in plasma, demonstrating that the drug concentration in the lungs is much higher compared to the systemic circulation. This indicates that inhalation provides a significantly higher drug concentration in the lungs, thereby potentially expanding the safety and efficacy window of EGCG for anti-pulmonary fibrosis.

Table 2: Cmax and AUC0-t of Plasma and Lung Drug Concentrations After Inhalation of 0.8 mg/kg and Oral Gavage of 60 mg/kg EGCG Solution.

| Administration method | Plasma $C_{max}$ (ng/mL) | Lung $C_{max}$ (ng/mL) | Plasma $AUC_{0-t}$ (h*ng/mL) | Lung $AUC_{0-t}$ (h*ng/mL) |
|---|---|---|---|---|
| oral gavage (po. 60mg/kg) | 46 | 34.5 | 136 | 115 |
| tracheal nebulization (inh.0.8mg/kg) | 153 | 12720 | 292 | 30891 |

Example 3: Pharmacokinetic Study of Low-Dose Tracheal Nebulization and Oral Gavage of EGCG in Rat Plasma and Lungs

As described in Example 1, the administration methods of oral gavage or tracheal nebulization of different doses of EGCG solution in rats were used to study the pharmacokinetic parameters of the drug in the lungs. The tracheal nebulization doses were 0.05 mg/kg, 0.1 mg/kg, 0.2 mg/kg, and 0.4 mg/kg, with 6-8 rats per dose group. The oral gavage

dose was 60 mg/kg, with 6 rats.

The blood sampling method and sample processing were as described in Example 2. The blood collection time points for this experiment were 5 minutes, 2 hours, 6 hours, 8 hours, 10 hours, and 16 hours post-administration, with approximately 3.5 mL of whole blood collected at each time point.

3.1 Experimental Results and Data Analysis:

After low-dose tracheal nebulization (doses of 0.05 mg/kg, 0.1 mg/kg, 0.2 mg/kg, and 0.4 mg/kg) and oral gavage (60 mg/kg) of EGCG solution, the changes in drug concentration in rat lungs over time are compared in Figure 4. From Figure 4, it can be seen that:

(1) When the inhalation dose is above 0.2 mg/kg, the maximum drug concentration (Cmax) in the lungs of rats is above 1 μg/mL (ranging from 1 to 20 μg/mL), while the lung concentration and plasma drug concentrations after oral gavage are both less than 100 ng/mL. This indicates that the drug concentration in the lungs is very high after nebulized inhalation.

(2) Even at a very low inhalation dose of 0.05 mg/kg, the lung drug concentration can reach levels comparable to those after oral gavage (60 mg/kg), where the inhalation dose is only 1/1200 of the oral dose. This shows that the inhalation route significantly expands the effective therapeutic dose window for EGCG.

(3) After tracheal nebulization of 0.1, 0.2, or 0.4 mg/kg EGCG solution, the drug concentration in the lungs can remain above the corresponding peak concentration (32 ng/mL) of the oral administration for up to 16 hours (greater than 50 ng/mL), indicating that tracheal nebulization allows the drug to maintain a high concentration level in the lungs for a long duration.

The average Cmax and AUC0-t in lung tissue after tracheal nebulization and oral gavage of EGCG solution are shown in Table 3. The average Cmax and AUC0-t data in Table 3 further demonstrate that EGCG solution, after nebulized inhalation, has excellent retention in the lungs, preventing rapid absorption into the systemic circulation post-administration, thereby greatly enhancing the local drug concentration in lung tissue. It can be inferred that the anti-IPF (idiopathic pulmonary fibrosis) activity of EGCG is likely driven by its high exposure in lung tissue. Thus, compared to oral administration, nebulized inhalation of EGCG significantly expands the safety and efficacy window, and inhalation delivery of EGCG offers the possibility of longer dosing intervals, enabling more flexible treatment regimens.

Table 3: Average Cmax and AUC0-t of Lung Tissue After Tracheal Nebulization and Oral Gavage of EGCG Solution.

| Administration method | Dosage mg/kg | Lung $C_{max}$ (ng/mL) | Lung $AUC_{0-t}$ (h*ng/mL) |
|---|---|---|---|
| oral gavage (po) | 60 | 43.4 | 226.4 |
| Tracheal Nebulization (inh) | 0.05 | 79.6 | 284.7 |
| | 0.1 | 220.4 | 1031.4 |
| | 0.2 | 1038.0 | 3082.7 |
| | 0.4 | 3180.0 | 11764.1 |

Example 4: Effect of Tracheal Nebulization of EGCG on Bleomycin-Induced Pulmonary Fibrosis in Mice

4.1 Experimental Animals

A total of 56 male C57BL/6J mice, SPF grade, aged 7-10 weeks, with a body weight range of 20-35g, were purchased from SPF (Beijing) Biotechnology Co., Ltd..

4.2 Main Reagents

Injectable Bleomycin (BLM, 15mg/vial, batch number 20067411): Hanhui Pharmaceutical Co., Ltd.

Solvent 1: Components include purified water, citric acid, and sodium citrate, a transparent, clear liquid.
Solvent 2: Sodium chloride injection (batch number K21080307), purchased from Hanhui Pharmaceuticals Co., Ltd (formerly Hisun-Pfizer Pharmaceuticals Co., Ltd.).
Hydroxyproline (HYP) assay kit: Purchased from Merck Sigma-Aldrich.

4.3 Animal Grouping and Model Construction

The mice were randomly divided into the following groups, with 8 mice in each group:

Sham operation group (normal control group),
Pulmonary fibrosis model group,
Oral gavage group,
Tracheal Nebulization groups 1-4.

On Day 1 (D1), the normal control group mice were administered solvent 2 (sodium chloride injection, 1 mL/kg) via tracheal nebulization. All other groups were administered bleomycin (2 mg/kg, 1 mL/kg) via tracheal nebulization in the morning and afternoon to construct the pulmonary fibrosis model.

4.4 Drug Dosage and Frequency

The oral gavage dose for mice was 100 mg/kg (calculated based on the oral dose of 60 mg/kg for rats, according to FDA guidelines, and converted for equivalent oral dosage in mice by body surface area). The tracheal nebulization doses were 0.2 mg/kg, 0.4 mg/kg, 0.8 mg/kg, and 1.6 mg/kg. Both the normal control group and the pulmonary fibrosis model group were administered solvent 1 via tracheal nebulization once a day, starting on D10 post-model induction.

4.5 Sample Collection

Mice from each group were anesthetized on Day 22 via intraperitoneal injection of chloral hydrate (50 mg/mL, 0.1 mL/10g) and sacrificed by abdominal aorta blood collection. All animals underwent dissection, and lung tissues were preserved. The total lung weight (including animals that died unexpectedly or were euthanized) was measured. The left lung was excised and stored at below -60°C for hydroxyproline content testing. The remaining right lung tissue and bronchioles were fixed in 10% neutral-buffered formalin and processed for routine histology: paraffin embedding, sectioning, HE staining, and Masson staining.

4.6 Detection Indicators

(1) Lung Coefficient:

$$\text{Lung coefficient} = \text{lung weight (mg)} / \text{body weight (g)}.$$

Early-stage inflammation with infiltration of inflammatory cells, followed by the proliferation of fibroblasts and excessive collagen deposition, leads to an increase in lung coefficient. Thus, the lung coefficient serves as an indirect indicator of the inflammation and fibrosis degree in mice.

(2) Mouse Body Weight:

Mice were weighed prior to grouping, on the model induction day, after the first drug administration, and every 3 days thereafter. Weighing was also performed before euthanasia or when any mouse showed signs of imminent death. Changes in body weight reflect the effect of the drug administration. Statistical analysis was performed using two-way ANOVA and Tukey's multiple comparison test.

(3) Pathological Examination:

Lung tissue inflammation, fibroproliferation, and other pathological changes were evaluated by optical microscopy on HE-stained sections. Masson staining was used to assess the degree of fibrosis in lung tissue. Standardized terminology was applied to classify fibrosis, inflammatory cell infiltration, alveolar hemorrhage, dilation, epithelial hyperplasia, and fibrous exudate using a 4-point scale (mild, moderate, severe, and very severe).

(4) Collagen Content in Lung Tissue:

Hydroxyproline content in lung tissue was measured according to the instructions of the assay kit.

(5) Statistical Analysis:

Statistical tests commonly used in the field, such as Student's t-test, chi-square test, Mann-Whitney U test, Kruskal-Wallis test (H test), Jonckheere-Terpstra test, and Wilcoxon test, were performed. Statistical analysis was done using SPSS (13.0) software. Results are presented as mean ± standard error. Overall differences were evaluated by One-Way ANOVA for homogeneity of variance, followed by Dunnett's test for inter-group comparisons. When normality tests failed, Kruskal-Wallis test was used for inter-group comparisons. Two-Way ANOVA and Tukey's test were used for multiple comparisons between groups. Statistically significant differences are indicated by asterisks (*$P < 0.05$; **$P < 0.01$; ***$P < 0.001$).

4.7 Experimental Results and Data Analysis

4.7.1 Effect of Inhalation Administration of EGCG on Lung Coefficient in Bleomycin-Induced Pulmonary Fibrosis Mice

Compared to the pulmonary fibrosis model group, the effect of tracheal nebulization of EGCG solution and oral gavage administration of EGCG solution on the lung weight index in mice is shown in Figure 5 (Kruskal-Wallis test, *: $p < 0.05$, indicating significant statistical difference; **: $p < 0.01$, indicating highly significant difference; ***: $p < 0.001$, indicating extremely significant difference). After model induction, the lung coefficient of the model group mice was significantly higher than that of the normal control group (**: $P < 0.01$). As shown in Figure 5, compared to the model group, the lung coefficient of mice in the tracheal nebulization groups and oral gavage group were significantly reduced, especially in the tracheal nebulization groups with doses of 0.2mg/kg and 0.4mg/kg, where the lung coefficient of disease mice was significantly reduced (**: $P < 0.01$; ***: $P < 0.001$). However, the oral gavage group did not show a significant difference compared to the model group.

4.7.2 Effect of Inhalation Administration of EGCG on Body Weight in Bleomycin-Induced Pulmonary Fibrosis Mice

Compared to the pulmonary fibrosis model group, the effect of tracheal nebulization of EGCG and oral gavage administration on the body weight of mice is shown in Figure 6 (statistical analysis was performed using Two-Way ANOVA and Tukey's multiple comparison test). As shown in Figure 6: (1) After bleomycin induction, the body weight of mice in all groups decreased significantly. (2) After oral gavage or tracheal nebulization of the experimental drug starting on Day 10, the body weight showed an upward trend, but the weight gain in the oral gavage group was slower, whereas the body weight of the 0.2mg/kg and 0.4mg/kg tracheal nebulization groups returned to baseline levels by Day 22, with significant differences compared to the model group (*$P < 0.05$; **$P < 0.01$; ***$P < 0.001$). This suggests that tracheal nebulization of appropriate doses of EGCG has less impact on the body weight of pulmonary fibrosis mice compared to oral gavage, and tracheal nebulization results in fewer side effects.

4.7.3 Effect of Inhalation Administration of EGCG Solution on Histopathological Changes in Lung Tissue of Bleomycin-Induced Pulmonary Fibrosis Mice

On D22, lung tissue sections from healthy control (control) group mice, pulmonary fibrosis model (model) group mice, oral gavage 100mg/kg (i.e., p.o. 100) EGCG group mice, and each dose tracheal nebulization group (inh. 0.2, inh. 0.4, inh. 0.8, inh. 1.6) mice were stained with Masson's and H&E staining, and the results are shown in Figures 7a and 7b.

As seen in Figures 7a and 7b: The lung tissue structure of healthy control (blank) group mice was clear, with uniform alveolar septa, no edema, no significant myofibroblasts, no inflammation, and no fibrosis. There was no obvious exudation in the alveolar cavity. The pulmonary fibrosis model (model) group mice exhibited severe alveolar structure destruction, atrophy and collapse, thickened alveolar septa, hyaline membrane formation, significant alveolar congestion, and extensive inflammatory cell infiltration around the small airways. Vascular endothelium was damaged, fibroblasts proliferated abnormally, collagen fibers were deposited significantly in the lung stroma, and the lung tissue became solid.

As shown in Figure 7a, Masson's staining results indicate that compared to the model group, tracheal nebulization of EGCG significantly reduced collagen deposition. There was a clear dose-response relationship in the different aerosol doses, with significant reductions in fibrosis, especially when doses of 0.4mg/kg and above were inhaled. The fibrosis in the lungs of mice after inhaling 0.8mg/kg and 1.6mg/kg was almost close to the level of the healthy control group. After oral administration of EGCG, collagen deposition in the lungs of mice decreased slightly, but the difference compared to the model group was not significant, and the effect was significantly weaker than tracheal nebulization. As shown in Figure 7b, H&E staining results show that after tracheal nebulization of EGCG, lung tissue exhibited reduced alveolar interstitial proliferation, and alveolar morphology was more complete, especially at the 0.4mg/kg and 0.8mg/kg doses, which showed the best results. After oral administration of EGCG, there was slight improvement in lung tissue morphology, but the effect was much weaker than tracheal nebulization, and no significant difference was observed

compared to the model group.

4.7.4 Effect of Inhalation Administration of EGCG Solution on Alveolar Inflammation and Pulmonary Fibrosis in Bleomycin-Induced Pulmonary Fibrosis Mice

Compared to oral gavage administration, tracheal nebulization of EGCG had a significant effect on alveolar inflammatory cell infiltration, hemorrhage, alveolar expansion, and fibrous exudates in the lungs of pulmonary fibrosis mice, as shown in Figure 8. The comparison of alveolar inflammation scores (Score) among the groups revealed the following:

1. Compared to the model group, tracheal nebulization of EGCG significantly suppressed the further development of alveolar inflammation in lung tissue. However, there was no significant difference between the oral gavage group and the model group, and there was even an increased trend in alveolar hemorrhage and alveolar epithelial hyperplasia, indicating that oral administration of EGCG was less effective in suppressing alveolar inflammation.
2. **In** terms of inhibiting alveolar expansion, all tracheal nebulization groups showed significant differences compared to the model group (inh. 1.6mg/kg: *P < 0.05; inh. 0.2mg/kg and inh. 0.8mg/kg: **P < 0.01; inh. 0.4mg/kg: ****P < 0.0001). The tracheal nebulization group at a dose of 0.4mg/kg showed the most effective result (****P < 0.0001).
3. **In** terms of inhibiting alveolar hemorrhage, the tracheal nebulization groups at doses of 0.2mg/kg and 0.4mg/kg were significantly better than the oral gavage group.
4. In terms of inhibiting alveolar fibrous exudates, the tracheal nebulization group at a dose of 1.6mg/kg showed significant differences compared to the model group.
5. In terms of inhibiting inflammatory cell infiltration, oral administration (e.g., oral gavage) of EGCG had no anti-inflammatory effect, while all tracheal nebulization groups reduced inflammatory cell infiltration, with the 0.4mg/kg tracheal nebulization group showing the best anti-inflammatory effect (score *: P < 0.05).

Compared to the pulmonary fibrosis model group, the effect of tracheal nebulization of EGCG and oral gavage administration on the fibrosis scores in mice is shown in Figure 9. As shown in the fibrosis degree score comparison in Figure 9, both the oral gavage group and the tracheal nebulization groups at all doses significantly reduced the fibrosis score. When the aerosol dose was 0.4mg/kg or higher, a significant reduction in pulmonary fibrosis was observed (score *P < 0.05), with the 0.2mg/kg, 0.4mg/kg, and 0.8mg/kg tracheal nebulization groups showing a good dose-response relationship. The oral gavage group did not show a significant difference compared to the model group.

These results indicate that tracheal nebulization of EGCG solution can produce anti-inflammatory effects at relatively low doses (0.2mg/kg). As the inhalation dose increases, the anti-fibrotic effects also gradually improve. The anti-fibrotic effect is especially evident at the 1.6mg/kg dose. Therefore, the effective dose range for inhalation of EGCG is quite broad.

4.7.5 Effect of Inhalation Administration of EGCG Solution on Hydroxyproline Content in Lung Tissue of Pulmonary Fibrosis Mice

The data on the effect of tracheal nebulization of EGCG and oral EGCG on hydroxyproline content in the lungs induced by bleomycin is shown in Table 4. The effect of oral gavage administration of EGCG and tracheal nebulization of EGCG on hydroxyproline content in lung tissue compared to the pulmonary fibrosis model group is shown in Figure 10 (analysis was performed using One-Way ANOVA and Dunnett's test for intergroup comparison). As shown in Table 4, on D 22, the hydroxyproline content in the lung tissue of pulmonary fibrosis mice significantly increased (***: P < 0.001). After tracheal nebulization of EGCG at doses of 0.2mg/kg, 0.4mg/kg, and 0.8mg/kg, the hydroxyproline content in lung tissue was dose-dependently reduced. Particularly at a dose of 0.8mg/kg, inhalation of EGCG significantly reduced hydroxyproline content in lung tissue, which was significantly better than oral administration (score ***: P < 0.001). The inhalation(such as tracheal nebulization) dose of 0.8mg/kg, as described in Example 2, showed similar plasma exposure to the oral dose of 60mg/kg in rats (equivalent to 100mg/kg in mice). One-Way ANOVA and Dunnett's test were used for intergroup comparison, with Student's T-test applied for statistical analysis where specified. *: P < 0.05, indicating significant difference; **: P < 0.01, indicating highly significant difference; ***: P < 0.001, indicating extremely significant difference.

Table 4: Tracheal Nebulization of EGCG vs. Oral EGCG on Hydroxyproline Content in Pulmonary Fibrosis Mice Induced by Bleomycin.

| Hydroxyproline Content (μg/μL) | Pulmonary fibrosis model group | P.O 100mg/kg | Inh. 0.2mg/kg | Inh. 0.4mg/kg | Inh. 0.8mg/kg | Inh. 1.6 mg/kg |
|---|---|---|---|---|---|---|
| Mean±SEM | 3.693 ±0.192 | 1.928 ±0.1326 | 2.807 ±0.3982 | 2.046 ±0.690 | 1.033 ±0.1476 | 1.67±0.1888 |

Example 5: Effect of Inhalation Administration of EGCG on Pulmonary Fibrosis Induced by Bleomycin in Rats

5.1 Research Objective

This study aims to compare the effects of different administration methods, including oral gavage of EGCG, tracheal nebulization of pirfenidone solution, and tracheal nebulization of EGCG at different doses, on the collagen content in lung tissues and pathological observations of rats with bleomycin-induced pulmonary fibrosis. The therapeutic effect of inhaled EGCG on rat pulmonary fibrosis was evaluated.

5.2 Main Reagents

The solvents for the EGCG nebulization solution, modeling agents, and their solvents are as described in Example 4. The positive control drug is pirfenidone (purity 99%, pharmaceutical grade), with the solvent components consisting of NaCl and citric acid, forming a clear liquid.

5.3 Model Construction and Grouping

SPF-grade SD rats, 6-9 weeks old with a weight of 200-300g, totaling 88 rats, were randomly divided into the following groups:

Sham operation group (normal control) (8 rats)
Pulmonary fibrosis model control group (10 rats)
Oral gavage EGCG group 1 (10 rats)
Oral gavage EGCG group 2 (10 rats)
Tracheal Nebulization EGCG solution groups 1-4 (10 rats per group, total 40 rats)
Tracheal Nebulization pirfenidone solution group (10 rats)

On D1, the normal control group was administered solvent 2 (saline injection solution, 1 mL/kg) via tracheal nebulization, while all other groups were administered bleomycin (2.5 mg/kg, 1 mL/kg) via tracheal nebulization twice a day (morning and afternoon) to establish the model. On D8, the body weights of the animals were recorded. From D10 to D28, the normal control and pulmonary fibrosis model groups were treated with solvent 1 via tracheal nebulization, while the remaining groups were administered EGCG or pirfenidone once daily at the following doses and administration methods:

Oral gavage EGCG group 1 and 2: 30 mg/kg, 60 mg/kg
Tracheal Nebulization EGCG group 1-4: 0.05 mg/kg, 0.4 mg/kg, 0.8 mg/kg, 1.6 mg/kg
Tracheal Nebulization pirfenidone solution group: 0.9 mg/kg (reference dose from M.W. Surber et al., ATS POSTER, 2014)

5.4 Sample Collection

On D29, all animals were euthanized for dissection. During the necropsy, the lungs, trachea, and bronchi were examined for abnormalities. Left lung tissue was preserved at below -60°C for hydroxyproline content detection, and right lung tissue was prepared for pathological sectioning. Hematoxylin and eosin (HE) staining and Masson staining were performed for pathological morphological observations and fibrosis evaluation.

5.5 Effect of Inhalation Administration of EGCG Solution on Hydroxyproline Content in Pulmonary Fibrosis Rat Lung Tissue

The data on the effect of oral gavage EGCG, tracheal nebulization EGCG solution, and tracheal nebulization pirfenidone solution on hydroxyproline content in bleomycin-induced rat lungs is shown in Table 5 and Figure 11.

All groups were compared to the model group using statistical analysis via Kruskal-Wallis test. The results are indicated as follows:

*P < 0.05: statistically significant difference
**P < 0.01: highly significant difference
***P < 0.001: extremely significant difference
****P < 0.0001: extremely highly significant difference

On D28, compared to the normal control group, the hydroxyproline content in the pulmonary fibrosis model group was significantly increased (****P < 0.0001). After oral administration of EGCG and tracheal nebulization of pirfenidone solution, the hydroxyproline content in rat lungs decreased, but the difference from the model group was not statistically significant. However, after inhalation of EGCG, the hydroxyproline content in rat lungs was significantly reduced (with statistical differences compared to the model group). Specifically:

Inhalation of EGCG at 0.4 mg/kg: ****P < 0.0001
Inhalation of EGCG at 0.8 mg/kg and 1.6 mg/kg: ***P < 0.001

Even at a very low dose of 0.05 mg/kg, inhalation EGCG significantly reduced hydroxyproline content in rat lung tissue (*P < 0.05). These results suggest that EGCG, when administered via inhalation, significantly reduces the degree of pulmonary fibrosis in the model animals, and the therapeutic effect window for effective doses is quite broad.

Table 5: Inhibition of Hydroxyproline Content in Bleomycin-Induced Rat Lungs by Oral Gavage EGCG, Tracheal Nebulization EGCG Solution, and Tracheal Nebulization Pirfenidone Solution.

| Hydroxyproline Content μg/μL | normal control | model control | PO 30mg/kg | PO 60mg/kg | Inh 0.05mg/kg | Inh 0.4mg/kg | Inh 0.8mg/kg | Inh 1.6 mg/kg | Positive control (AP01) Inh 0.9 mg/kg |
|---|---|---|---|---|---|---|---|---|---|
| Mean ±SEM | 0.407 ±0.047 | 0.702 ±0.036 | 0.608 ±0.012 | 0.546 ±0.026 | 0.528 ±0.038 | 0.433 ±0.023 | 0.485 ±0.027 | 0.465 ±0.028 | 0.587 ±0.059 |

Example 6: Preparation of EGCG Nebulized Solution

6.1 Formulation

| | |
|---|---|
| EGCG: | 10g |
| Citric acid: | Appropriate amount |
| Sodium citrate: | Appropriate amount |
| Water for injection: | 1000mL |

6.2 Preparation Method

Mix the EGCG raw material with the excipients in a mixing tank, then add water for injection up to 1000mL. Stir to dissolve and cool. Next, adjust the pH of the solution to 3-4 using citric acid and sodium citrate. After prefiltering the EGCG solution, perform sterile filtration and fill the solution aseptically to obtain the EGCG nebulized solution.

In another method for preparing EGCG nebulized solution, the formulation differs from that in 6.1 by also containing an appropriate amount of EDTA, with the preparation method the same as described in 6.2.

Example 7: Single-Dose Escalation Study of EGCG Nebulized Inhalation Solution in Healthy Volunteers

A randomized, double-blind, placebo-controlled clinical study was conducted with 22 healthy volunteers to evaluate the safety and tolerability of single-dose inhalation of EGCG nebulized solution, as well as pharmacokinetic characteristics, compared to placebo nebulized solution and oral EGCG capsules.

**Test Drugs:**

Test group: EGCG nebulized inhalation solution (the EGCG nebulized solution containing EDTA as described in Example 6), with an initial concentration of 10mg/mL, diluted to different doses before administration.

Placebo group: Blank formulation

Oral control group: EGCG capsules, each weighing 150mg, with EGCG content approximately 94% of the total weight.

**Groupings and Administration:**

Refer to Table 6 below. The oral control group was randomly assigned 4 volunteers to receive 600mg of EGCG capsules (4 capsules, approximately 564mg of EGCG).
The EGCG nebulized inhalation treatment included 3 groups:

1. 3mg dose group: 1mL of the starting drug solution was diluted to 1mg/mL, and 3mL was administered via the nebulizer.
2. 10mg dose group: 1mL of the starting drug solution was diluted to 3.33mg/mL, and 3mL was administered via the nebulizer.
3. 30mg dose group: The starting drug solution was not diluted, and 3mL was directly administered via the nebulizer.

Each nebulized inhalation dose group was randomly assigned 6 volunteers, starting with the lowest dose. In each group, 2 volunteers were designated as sentinels (1:1 randomization, 1 in the EGCG nebulized inhalation group and 1 in the placebo group), observed for ≥24 hours, and the study investigator performed a safety assessment. After permission from the investigator, the remaining 4 volunteers in each group received their assigned treatment (3:1 randomization, 3 in the EGCG nebulized inhalation group and 1 in the placebo group).

Table 6: Group Information for Oral Control and EGCG Nebulized Inhalation Administration Groups.

| Group | Oral control group | EGCG nebulized inhalation dose group: EGCG nebulized inhalation solution 3mg/10mg/30mg | | | |
|---|---|---|---|---|---|
| | | Sentinel group | | Dose group | |
| | | EGCG nebulized inhalation solution | Placebo | EGCG nebulized inhalation solution | Placebo |
| Number of subjects administered | 4 | 1 | 1 | 3 | 1 |

**Blood sample collection:**

Blood samples were collected from the subjects at 13 time points: pre-dose (within 1 hour before administration), and post-dose at 15 minutes, 30 minutes, 1 hour, 1.5 hours, 2 hours, 3 hours, 4 hours, 6 hours, 8 hours, 10 hours, 12 hours, and 24 hours. A total of 4mL of blood was collected at each time point, and plasma was extracted for pharmacokinetic (PK) testing, backup, and metabolite identification.

**Safety assessment:**

Adverse event (AE) monitoring; physical examination; vital signs (body temperature, blood pressure, pulse, respiration), blood oxygen saturation; heart function; lung function.

**Inclusion criteria for participant screening:**

The research subjects should meet the following criteria:

- Healthy adult subjects aged 18-59 years (inclusive of both ends), regardless of gender.
- Understand the research procedures and methods, voluntarily participate in the trial, and sign a written informed consent.
- Body Mass Index (BMI = weight/height$^2$ (kg/m$^2$)): $18 \leq$ BMI < 28; male weight $\geq$ 50.0kg and < 90.0kg, female weight $\geq$ 45.0kg and < 90.0kg.
- No clinically significant abnormalities confirmed through medical history, physical examination, laboratory tests, vital signs, or ECG, and deemed medically healthy by the investigator.
- Agree to take effective contraceptive measures during the entire study period. Females of childbearing potential should have been using contraception for at least one month before screening and agree to continue contraception throughout the study and for 3 months after the study ends. Male subjects agree to use contraception during the entire study and for 3 months after the study ends and ensure no sperm donation.
- Forced Expiratory Volume in 1 second (FEV1) and Forced Vital Capacity (FVC) $\geq$ 80% of the predicted value, FEV1/FVC $\geq$ 0.7, and normal chest X-ray.
- Agree not to consume any coffee, tea, or beverages/foods containing coffee or tea ingredients 48 hours before and 48 hours after administration.
- Able to correctly and effectively use the inhaler during the screening and study period.
- No use of any drugs 7 days before administration and no use of other drugs within 24 hours after administration.

**Exclusion criteria for participant screening:**

Subjects meeting any of the following criteria will be excluded from the study:

- Significant medical history that the investigator deems may have an adverse effect on participation.
- Female subjects who are pregnant or breastfeeding.
- Female subjects of childbearing potential who have not used contraception for at least 30 days prior to administration, or male subjects unwilling to use contraception or guarantee no sperm donation during the study and for 90 days after administration, or females of childbearing potential who do not agree to use contraception.
- Allergy to the study drug or any component of the study drug.
- Positive test results for COVID-19, HIV, Hepatitis B, or Hepatitis C.
- Subjects with alanine aminotransferase (ALT), aspartate aminotransferase (AST), or gamma-glutamyl-transferase (GGT) levels above the upper limit of normal (ULN), or total bilirubin above the ULN during screening/baseline visits.
- Smoking or using e-cigarettes within the past 6 months before the first dose.
- History of drug abuse or alcohol abuse within the past 3 months before screening, where alcohol abuse is defined as more than 21 alcohol units per week (1 unit = 284mL of beer, 25mL of 40% spirits, or 125mL of wine).
- Blood/plasma donation $\geq$ 400mL within 3 months before administration.

**Trial Results:**

No significant abnormalities were observed in physical examination, vital signs, heart function, and lung function of all subjects. Only a few subjects in the placebo group and the nebulized drug group experienced local symptoms in the oropharynx and respiratory tract, including throat itching, dry mouth, bitter taste, and coughing. These symptoms were mild and disappeared after stopping the medication: in the 10mg dose group, 1 out of 4 subjects had symptoms, and in the placebo group, 1 out of 2 subjects had symptoms; in the 30mg dose group, 2 out of 4 subjects had symptoms; no subjects in the 3mg dose group experienced these symptoms. The investigator concluded that after EGCG nebulized inhalation, the overall tolerability was good, and the safety profile was high.

The average plasma concentration-time curves of EGCG in each group post-administration are shown in Figure 12. As seen in the experimental results from Figure 12, a good correlation was observed between the drug exposure in plasma and the administration dose after inhalation. The difference in plasma drug exposure between inhalation and oral administration was much higher than the difference between the oral and inhaled doses.

In general, compared to oral administration, the absorption rate of pulmonary drug delivery is faster, with the drug quickly entering the systemic circulation through highly permeable pulmonary capillaries and alveolar surfaces, rapidly reaching a high peak. However, surprisingly, after EGCG inhalation, the time to reach peak plasma concentration (Tmax) was later, and the Cmax level was lower. Despite achieving the same or better pulmonary exposure as oral administration and producing equivalent or better therapeutic effects, the plasma concentration of the drug after inhalation was much

lower than after oral administration. For instance, in the 10mg dose group, the inhalation dose was 1/56 of the oral dose, while the inhalation Cmax was about 1/227 of the oral Cmax. This also suggests that EGCG nebulized inhalation could significantly increase the drug's retention in the lungs and its residence time, greatly reducing its concentration in plasma. The above results indicate that EGCG nebulized inhalation can significantly enhance the drug's therapeutic effect in the lungs while greatly reducing the risk of systemic side effects.

In conclusion, the application of (-)-epigallocatechin gallate (EGCG) compounds provided in this disclosure involves direct pulmonary delivery of EGCG via inhalation, increasing drug exposure in the lung tissue. This increases the concentration ratio of the drug in the lungs to plasma, enhancing efficacy while reducing potential hepatotoxicity. Even at very low inhaled doses, significant anti-inflammatory and anti-pulmonary fibrosis effects can be achieved, significantly expanding the safety treatment window of EGCG for IPF. After inhaled administration of EGCG, the drug can be maintained in the lungs at a high effective concentration for a long time, which is higher than the concentration achievable in lung tissue after oral administration, offering the potential to reduce administration frequency, increase patient compliance, and enable individualized dosing regimens. Moreover, inhalation avoids the issue of drug-drug interactions between oral EGCG and other IPF treatments.

It should be understood that the application provided herein is not limited to the above examples. Those skilled in the art may make improvements or modifications based on the above descriptions, and all such improvements and modifications should fall within the scope of the claims appended to this disclosure.

## Claims

1. An inhalable pharmaceutical composition comprising (-)-epigallocatechin gallate compound for use in the prevention and/or treatment of pulmonary fibrosis, wherein the (-)-epigallocatechin gallate compound is (-)-epigallocatechin gallate or a pharmaceutically acceptable salt, ester, hydrate, or solvate thereof.

2. The inhalable pharmaceutical composition comprising (-)-epigallocatechin gallate compound for use according to claim 1, wherein the inhalable pharmaceutical composition is formulated into an inhalable dosage form, which is selected from a solution, suspension, aerosol, or dry powder inhalation.

3. The inhalable pharmaceutical composition comprising (-)-epigallocatechin gallate compound for use according to claim 1, wherein the inhalable pharmaceutical composition is prepared by processing the (-)-epigallocatechin gallate compound into a powder through a drying process, which is then redissolved with a diluent.

4. The inhalable pharmaceutical composition comprising (-)-epigallocatechin gallate compound for use according to claim 2, wherein when the dosage form is a solution, the inhalable pharmaceutical composition comprises the (-)-epigallocatechin gallate compound as the active ingredient, diluents, and one or more selected from pH modifiers, osmotic pressure adjusters, and antioxidants, wherein the (-)-epigallocatechin gallate compound is completely dissolved in the diluent;

   When the dosage form is a suspension, the inhalable pharmaceutical composition comprises the (-)-epigallocatechin gallate compound as the active ingredient, diluents, and one or more selected from surfactants, **pH** modifiers, and tension regulators, wherein the (-)-epigallocatechin gallate compound combines with pharmaceutically acceptable carriers for inhalation administration, forming particles that are suspended in a diluent;
   When the dosage form is an aerosol, the inhalable pharmaceutical composition comprises the (-)-epigallocatechin gallate compound as the active ingredient, diluents, propellants, and one or more selected from surfactants, co-solvents, and pH modifiers;
   When the dosage form is a dry powder inhalation, the inhalable pharmaceutical composition comprises the (-)-epigallocatechin gallate compound as the active ingredient, pharmaceutically acceptable carriers for inhalation administration, and one or more selected from excipients and surfactants.

5. The inhalable pharmaceutical composition comprising (-)-epigallocatechin gallate compound for use according to claim 3 or 4, wherein at least one of the following conditions (1) - (3) is satisfied:

   (1) The diluent is one or more selected from water, ethanol, and glycerol;
   (2) The pH of the inhalable pharmaceutical composition ranges from 3.0 to 5.0;
   (3) The concentration of the (-)-epigallocatechin gallate compound in the inhalable pharmaceutical composition ranges from 0.1 mg/mL to 25 mg/mL.

6. The inhalable pharmaceutical composition comprising (-)-epigallocatechin gallate compound for use according to claim 1, wherein the inhalable pharmaceutical composition comprises the (-)-epigallocatechin gallate compound, or the inhalable pharmaceutical composition comprises the (-)-epigallocatechin gallate compound and other anti-pulmonary fibrosis drugs.

7. The inhalable pharmaceutical composition comprising (-)-epigallocatechin gallate compound for use according to claim 6, wherein the other anti-pulmonary fibrosis drugs are selected from one or more of pirfenidone, nintedanib, glucocorticoids, immunosuppressants, prostacyclin and its analogs, CTGF antibodies, Galectin-3 inhibitors, integrin antagonists, recombinant serum amyloid P and its analogs, PDE inhibitors, LPA antagonists, JAK kinase inhibitors, and various cytokine receptor TKIs.

8. The inhalable pharmaceutical composition comprising (-)-epigallocatechin gallate compound for use according to claim 7, wherein the other anti-pulmonary fibrosis drugs are selected from one or more of pirfenidone, nintedanib, BI 1015550, treprostinil and its analogs, recombinant serum amyloid P, and LPA antagonists.

9. The inhalable pharmaceutical composition comprising (-)-epigallocatechin gallate compound for use according to claim 1, wherein the administered dose of the (-)-epigallocatechin gallate compound ranges from 0.1 mg to 100 mg per administration.

10. The inhalable pharmaceutical composition comprising (-)-epigallocatechin gallate compound for use according to claim 9, wherein the administered dose of the (-)-epigallocatechin gallate compound ranges from 0.1 mg to 50 mg per administration.

11. The inhalable pharmaceutical composition comprising (-)-epigallocatechin gallate compound for use according to claim 10, wherein the administered dose of the (-)-epigallocatechin gallate compound ranges from 0.1 mg to 30 mg per administration.

12. The inhalable pharmaceutical composition comprising (-)-epigallocatechin gallate compound for use according to claim 11, wherein the administered dose of the (-)-epigallocatechin gallate compound ranges from 0.1 mg to 15 mg per administration.

13. The inhalable pharmaceutical composition comprising (-)-epigallocatechin gallate compound for use according to claim 9, wherein the administered dose of the (-)-epigallocatechin gallate compound is selected from 0.1 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 25 mg, 30 mg, 35 mg, 40 mg, 45 mg, 50 mg, 55 mg, 60 mg, 65 mg, 70 mg, 75 mg, 80 mg, 90 mg, or 100 mg per administration.

14. The inhalable pharmaceutical composition comprising (-)-epigallocatechin gallate compound for use according to claim 1, wherein the administered dose of the (-)-epigallocatechin gallate compound ranges from 1/1000 to 1/10 of the oral dose.

15. The inhalable pharmaceutical composition comprising (-)-epigallocatechin gallate compound for use according to claim 14, wherein the dosage of the (-)-epigallocatechin gallate compound ranges from 1/600 to 1/10 of the oral dose.

16. The inhalable pharmaceutical composition comprising (-)-epigallocatechin gallate compound for use according to any one of claims 1-15, wherein the pulmonary fibrosis is an interstitial lung disease, including one or more of idiopathic interstitial pneumonia, pulmonary interstitial fibrosis caused by autoimmune or connective tissue diseases, exposure- or occupation-related interstitial fibrosis, treatment-induced pulmonary interstitial fibrosis, and sarcoidosis.

17. The inhalable pharmaceutical composition comprising (-)-epigallocatechin gallate compound for use according to claim 16, wherein the idiopathic interstitial pneumonia includes idiopathic pulmonary fibrosis;

The pulmonary interstitial fibrosis caused by autoimmune or connective tissue diseases includes lupus, scleroderma, polymyositis or dermatomyositis, and rheumatoid arthritis-associated interstitial lung disease;
The exposure- or occupation-related interstitial fibrosis includes asbestosis, silicosis, and hypersensitivity pneumonitis;
The treatment-induced pulmonary interstitial fibrosis includes interstitial lung diseases caused by chemotherapy,

radiotherapy, and certain drug treatments.

**18.** An inhalable pharmaceutical composition for preventing and/or treating pulmonary fibrosis, comprising (-)-epigallocatechin gallate compound as an active ingredient and pharmaceutically acceptable excipients; wherein the (-)-epigallocatechin gallate compound is (-)-epigallocatechin gallate or a pharmaceutically acceptable salt, ester, hydrate, or solvate thereof.

**19.** The pharmaceutical composition according to claim 18, wherein the content of the (-)-epigallocatechin gallate compound in each dose of the inhalable pharmaceutical composition ranges from 0.1 mg to 50 mg.

**20.** The pharmaceutical composition according to claim 19, wherein the content of the (-)-epigallocatechin gallate compound in each dose of the inhalable pharmaceutical composition ranges from 0.5 mg to 30 mg.

**21.** The pharmaceutical composition according to any one of claims 18-20, wherein the dosage form of the inhalable pharmaceutical composition is an inhalation solution, inhalation suspension, aerosol, or dry powder inhalation.

**22.** The pharmaceutical composition according to claim 21, wherein:

When the dosage form of the inhalable pharmaceutical composition is an inhalation solution, the pharmaceutically acceptable excipients are selected from one or more of surfactants, pH modifiers, antioxidants, preservatives, osmotic pressure regulators, metal ion chelating agents, water, and additives.
When the dosage form of the inhalable pharmaceutical composition is an inhalation suspension, the pharmaceutically acceptable excipients are selected from one or more of surfactants, pH modifiers, antioxidants, preservatives, osmotic pressure regulators, metal ion chelating agents, water, and additives.
When the dosage form of the inhalable pharmaceutical composition is an aerosol, the pharmaceutically acceptable excipients are selected from one or more of cosolvents, surfactants, propellants, and additives.
When the dosage form of the inhalable pharmaceutical composition is a dry powder inhalation, the pharmaceutically acceptable excipients include excipients, carriers, and additives.

**23.** A method for preventing and/or treating pulmonary fibrosis, wherein the method comprises administering a therapeutically effective amount of (-)-epigallocatechin gallate compound to subjects via inhalation, wherein the (-)-epigallocatechin gallate compound is (-)-epigallocatechin gallate or a pharmaceutically acceptable salt, ester, hydrate, or solvate thereof.

**24.** The method according to claim 23, wherein the administered dose of the (-)-epigallocatechin gallate compound ranges from 0.01 mg/kg to 2.0 mg/kg.

**25.** The method according to claim 23, wherein the administered dose of the (-)-epigallocatechin gallate compound ranges from 0.1 mg to 80 mg per administration.

**26.** The method according to claim 25, wherein the administered dose of the (-)-epigallocatechin gallate compound ranges from 0.1 mg to 50 mg per administration.

**27.** The method according to claim 26, wherein the administered dose of the (-)-epigallocatechin gallate compound ranges from 0.1 mg to 30 mg per administration.

**28.** The method according to claim 27, wherein the administered dose of the (-)-epigallocatechin gallate compound ranges from 0.1 mg to 15 mg per administration.

**29.** The method according to claim 25, wherein the administered dose of the (-)-epigallocatechin gallate compound is selected from 0.1 mg, 1 mg, 1.5 mg, 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, 9.5 mg, 10 mg, 10.5 mg, 11 mg, 11.5 mg, 12 mg, 12.5 mg, 13 mg, 13.5 mg, 14 mg, 14.5 mg, 15 mg, 15.5 mg, 16 mg, 16.5 mg, 17 mg, 17.5 mg, 18 mg, 18.5 mg, 19 mg, 19.5 mg, 20 mg, 25 mg, 30 mg, 35 mg, 40 mg, 45 mg, 50 mg, 55 mg, 60 mg, 65 mg, 70 mg, 75 mg, 80 mg, 90 mg, or 100 mg per administration.

**30.** The method according to claim 23, wherein the administered dose of the (-)-epigallocatechin gallate compound ranges from 1/1000 to 1/10 of the oral dose.

**31.** The method according to claim 30, wherein the administered dose of the (-)-epigallocatechin gallate compound ranges from 1/600 to 1/10 of the oral dose.

**32.** The method according to claim 23, further comprising administering another anti-pulmonary fibrosis drug to the subject via inhalation, wherein the other anti-pulmonary fibrosis drug is administered simultaneously, separately, or sequentially with the (-)-epigallocatechin gallate compound.

**33.** The method according to claim 32, wherein the other anti-pulmonary fibrosis drug is selected from one or more of pirfenidone, nintedanib, glucocorticoids, immunosuppressants, prostacyclin and its analogs, CTGF antibodies, Galectin-3 inhibitors, integrin antagonists, recombinant serum amyloid P and its analogs, PDE inhibitors, LPA antagonists, JAK kinase inhibitors, and multiple cytokine receptor TKIs.

**34.** The method according to claim 33, wherein the other anti-pulmonary fibrosis drug is selected from one or more of pirfenidone, nintedanib, BI 1015550, treprostinil and its analogs, recombinant serum amyloid P, and LPA antagonists.

**35.** The method according to any one of claims 23-34, wherein the (-)-epigallocatechin gallate compound is administered at a dosing regimen of once every 48 h, once daily, or twice daily.

**36.** The method according to any one of claims 23-34, wherein inhalation administration is performed via an inhalation delivery device.

**37.** The method according to claim 36, wherein the inhalation delivery device is a nebulizer, a pressurized metered-dose inhaler, a powder inhaler, or a soft mist inhaler.

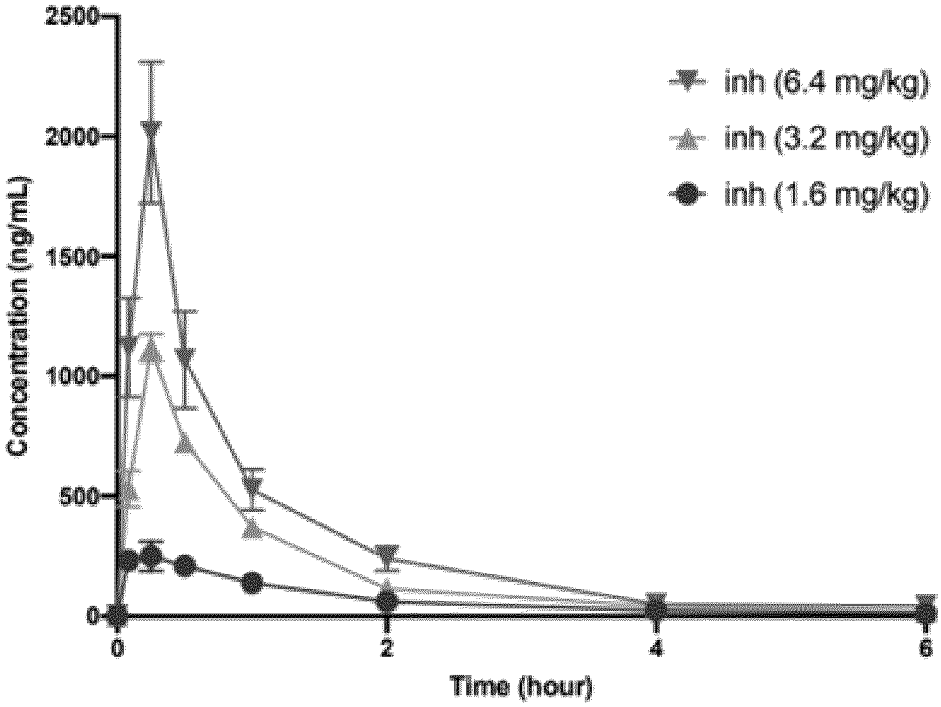
2500
2000
1500
1000
500
0
Concentration (ng/mL)
0
2
4
6
Time (hour)
inh (6.4 mg/kg)
inh (3.2 mg/kg)
inh (1.6 mg/kg)

Fig. 1

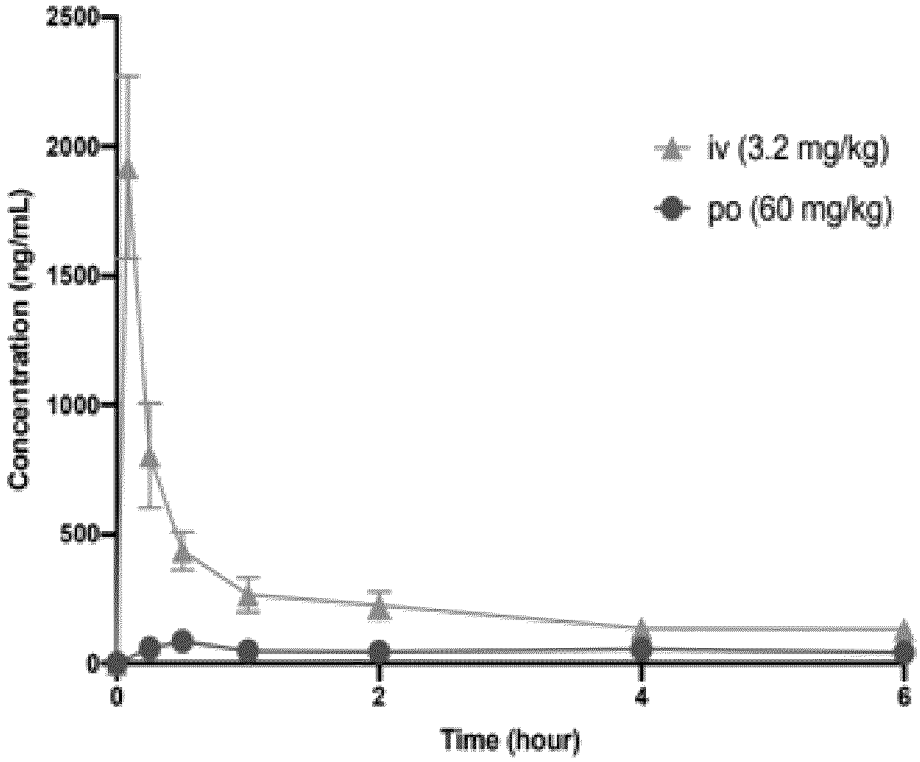
2500
2000
1500
1000
500
0
Concentration (ng/mL)
0
2
4
6
Time (hour)
iv (3.2 mg/kg)
po (60 mg/kg)

Fig. 2

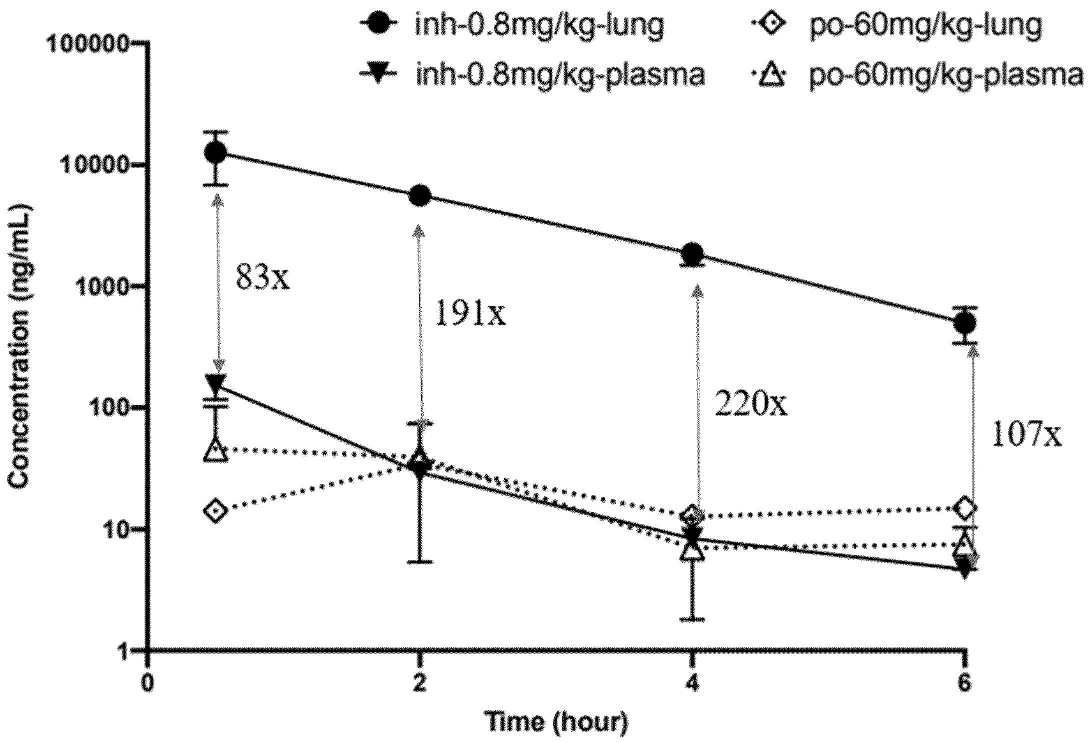
inh-0.8mg/kg-lung
inh-0.8mg/kg-plasma
po-60mg/kg-lung
po-60mg/kg-plasma
Concentration (ng/mL)
Time (hour)
83x
191x
220x
107x
100000
10000
1000
100
10
1
0
2
4
6

Fig. 3

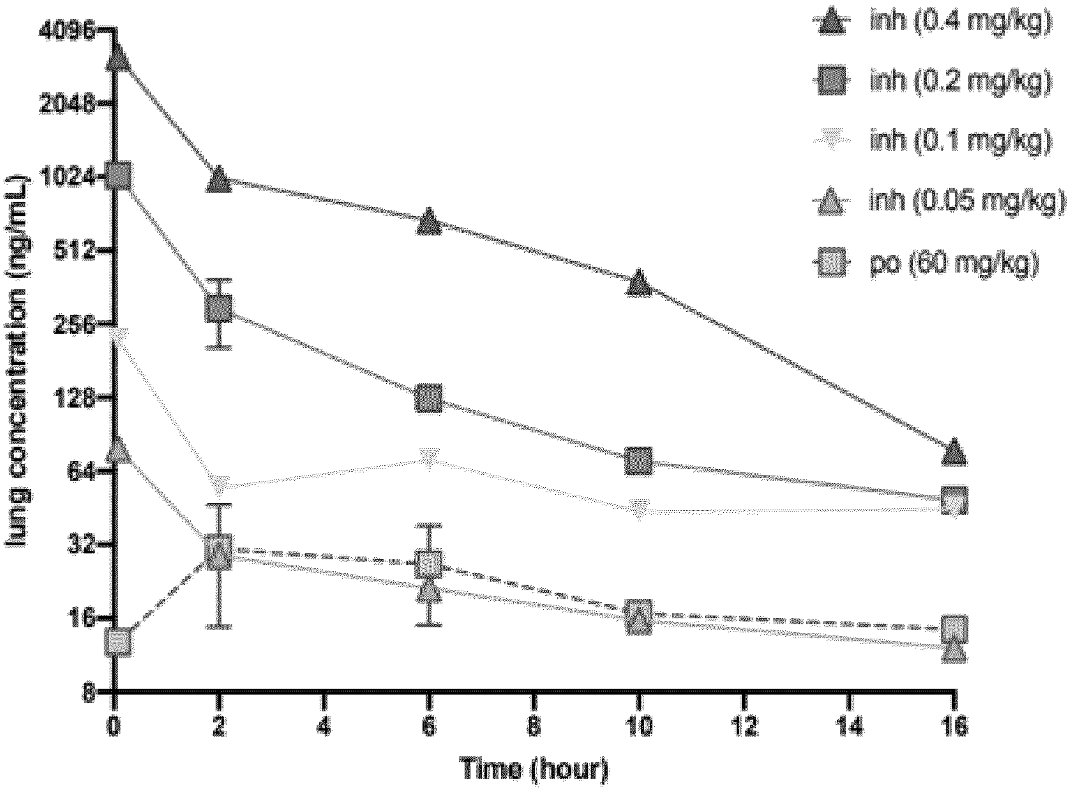
inh (0.4 mg/kg)
inh (0.2 mg/kg)
inh (0.1 mg/kg)
inh (0.05 mg/kg)
po (60 mg/kg)
lung concentration (ng/mL)
Time (hour)
4096
2048
1024
512
256
128
64
32
16
8
0
2
4
6
8
10
12
14
16

Fig. 4

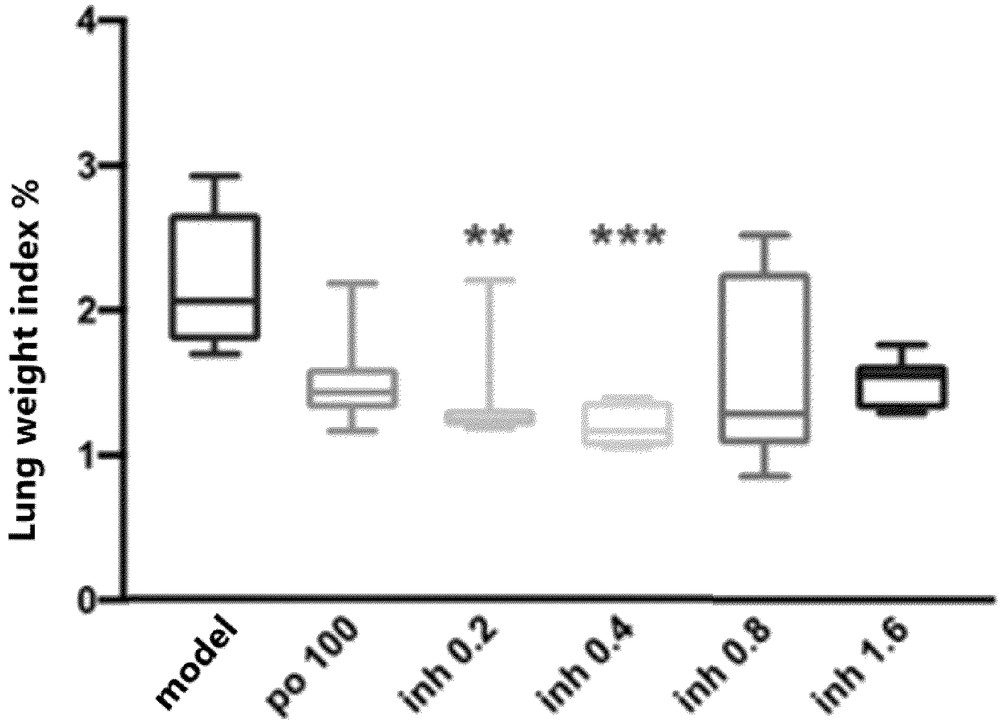
Lung weight index %
0
1
2
3
4
**
***
model
po 100
inh 0.2
inh 0.4
inh 0.8
inh 1.6

Fig. 5

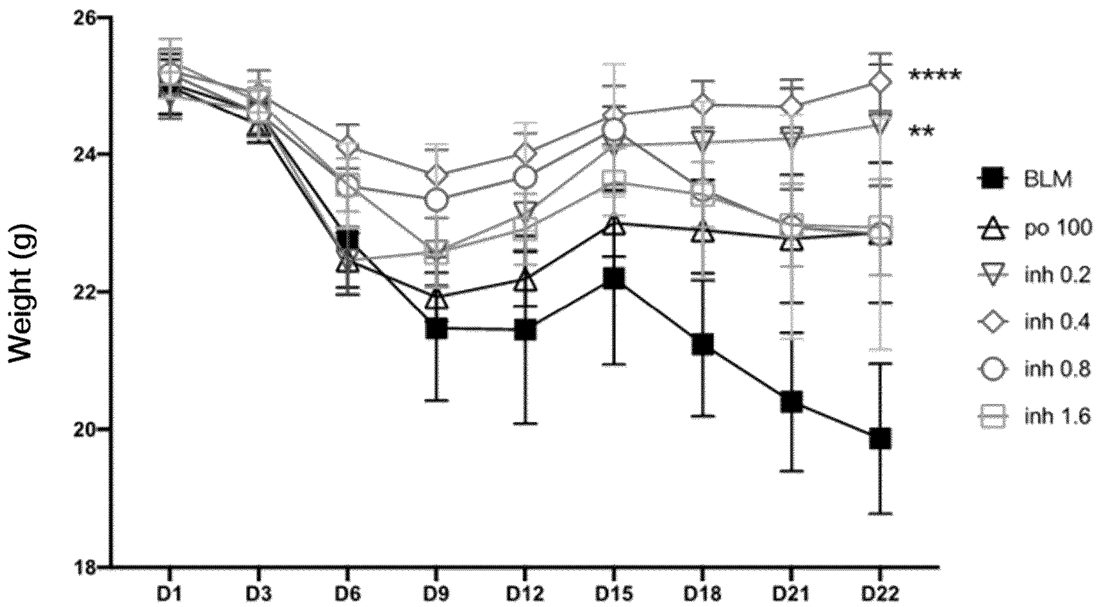
Weight (g)
18
20
22
24
26
D1
D3
D6
D9
D12
D15
D18
D21
D22
****
**
BLM
po 100
inh 0.2
inh 0.4
inh 0.8
inh 1.6

Fig. 6

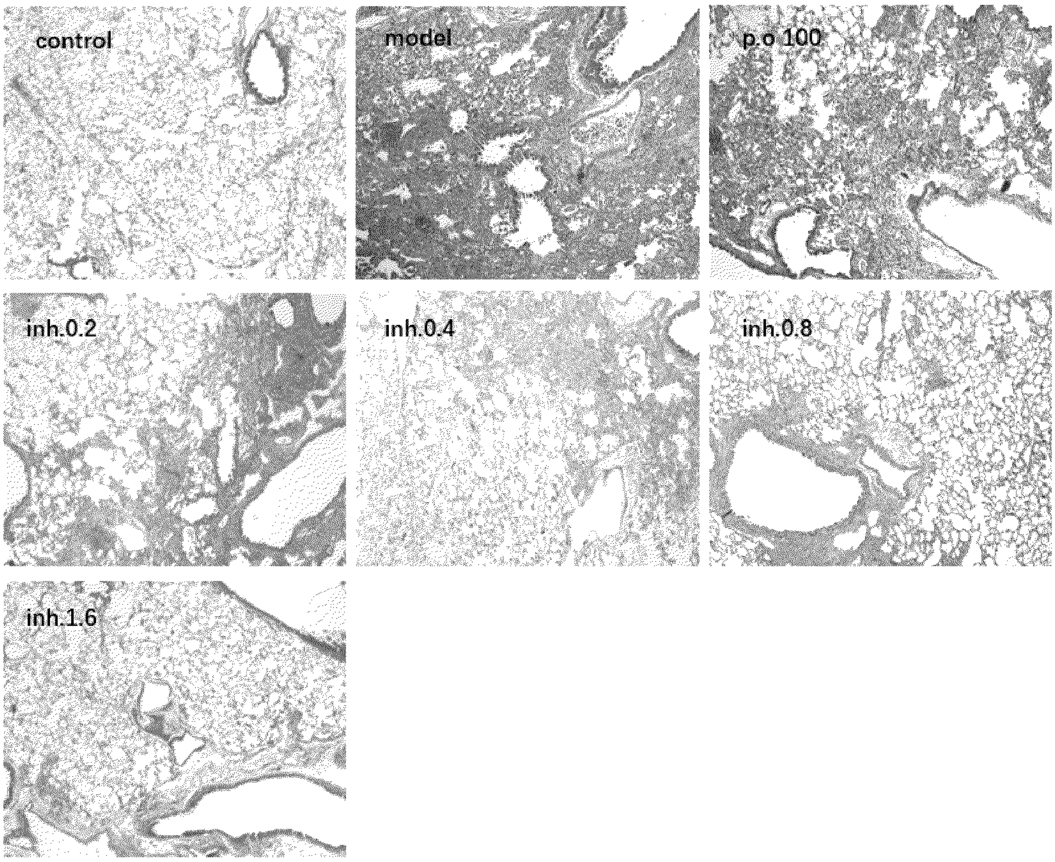
control
model
p.o 100
inh.0.2
inh.0.4
inh.0.8
inh.1.6

Fig. 7A

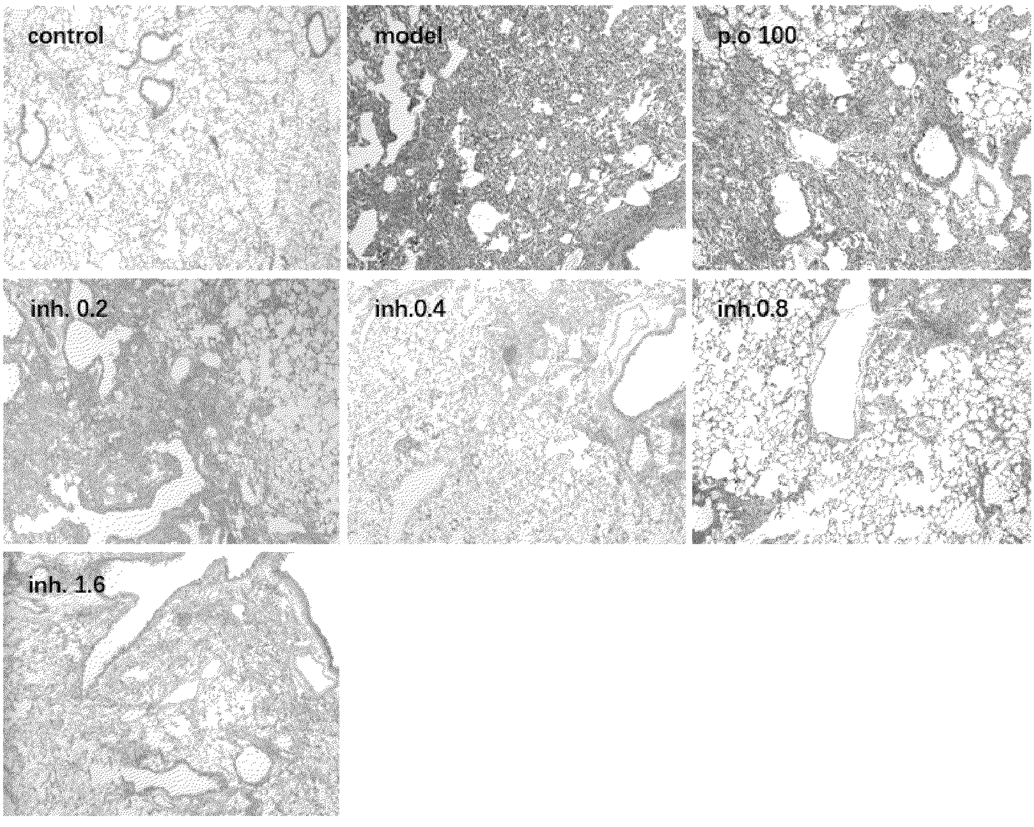
control
model
p.o 100
inh. 0.2
inh.0.4
inh.0.8
inh. 1.6

Fig. 7B

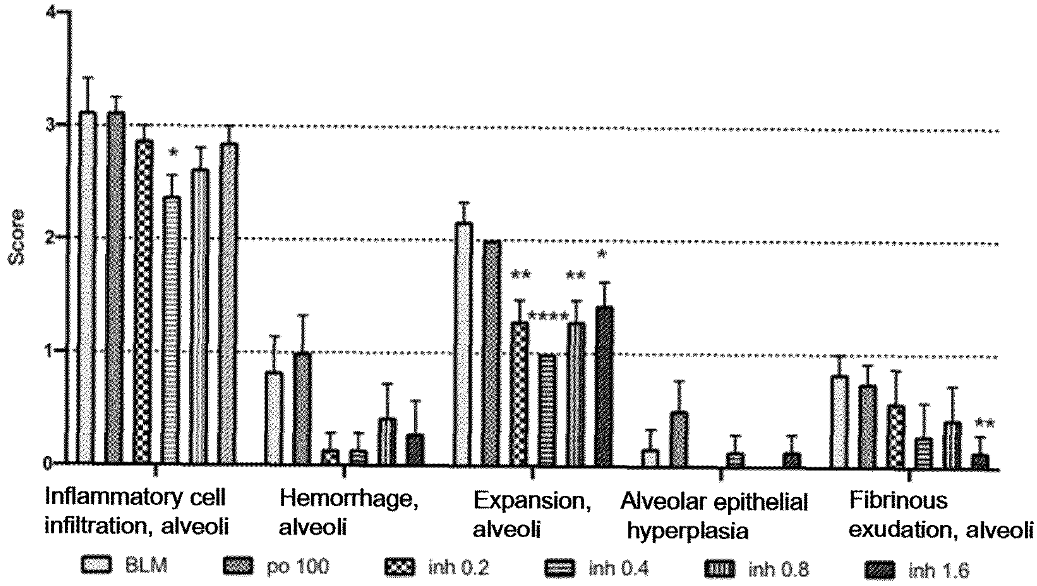


Fig. 8

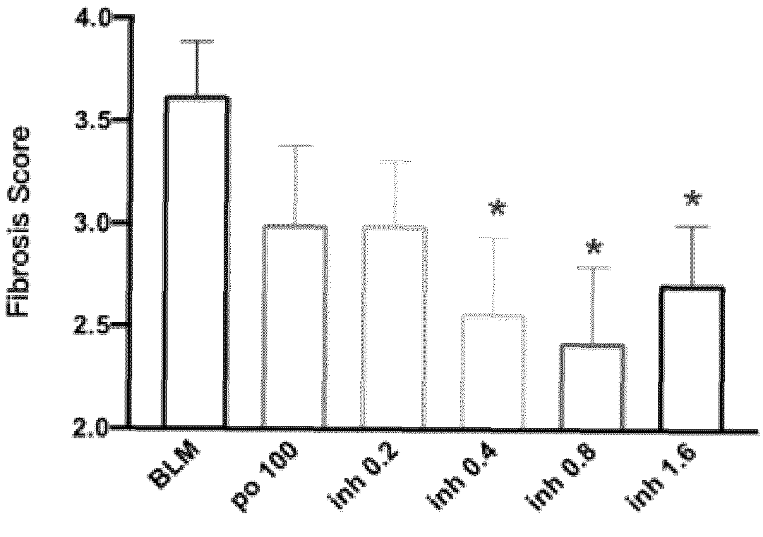


Fig. 9

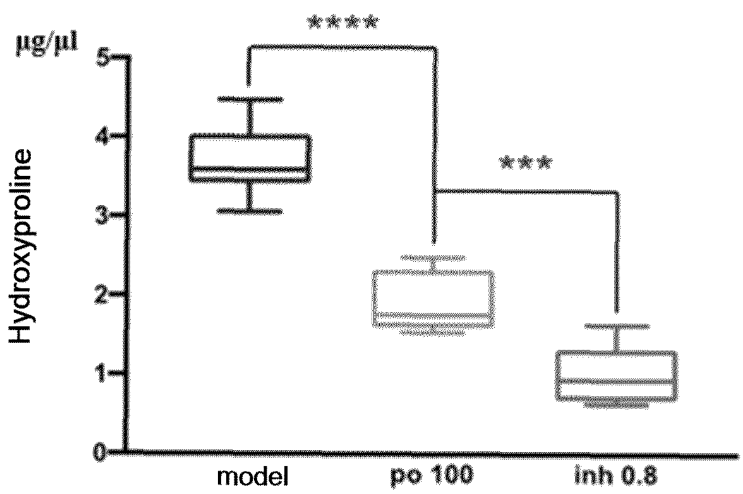


Fig. 10

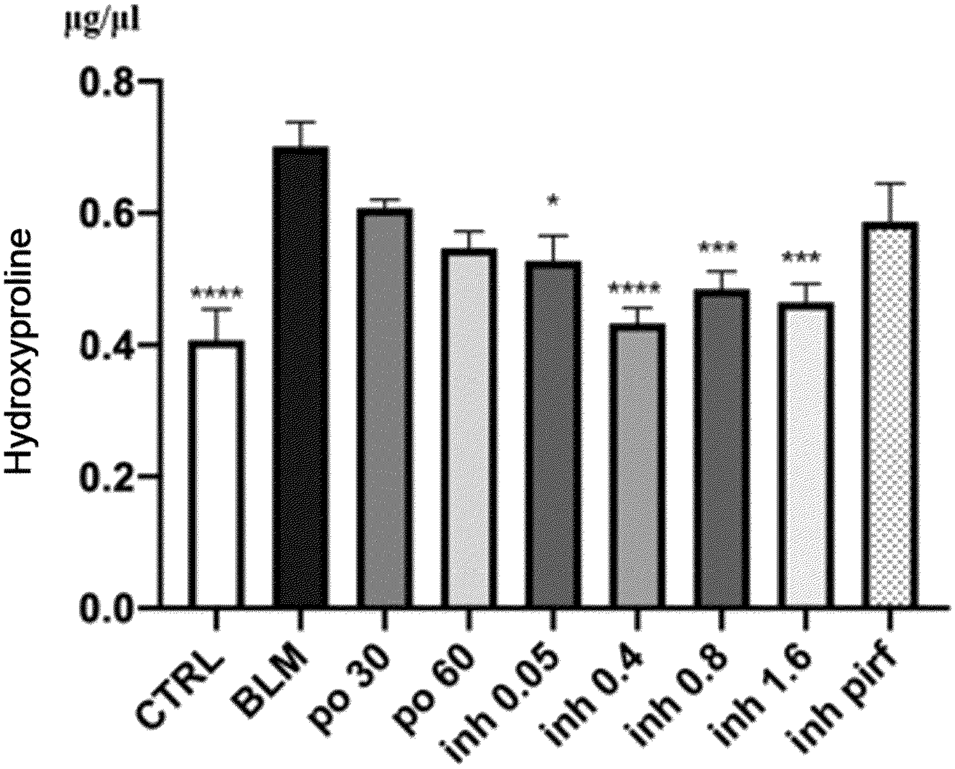
µg/µl
Hydroxyproline
0.8
0.6
0.4
0.2
0.0
****
*
****
***
***
CTRL
BLM
po 30
po 60
inh 0.05
inh 0.4
inh 0.8
inh 1.6
inh pirf

Fig. 11

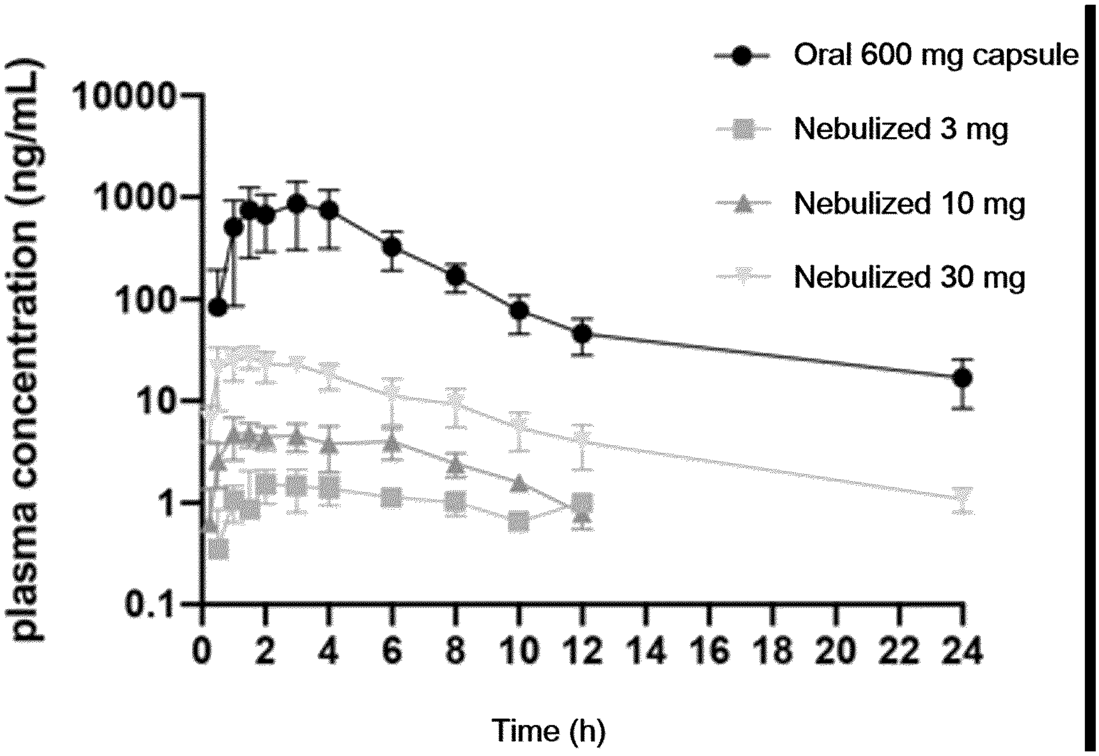
Oral 600 mg capsule
Nebulized 3 mg
Nebulized 10 mg
Nebulized 30 mg
plasma concentration (ng/mL)
10000
1000
100
10
1
0.1
0
2
4
6
8
10
12
14
16
18
20
22
24
Time (h)

Fig. 12

## INTERNATIONAL SEARCH REPORT

International application No.
**PCT/CN2023/132299**

**A. CLASSIFICATION OF SUBJECT MATTER**

A61K31/353(2006.01)i； A61K9/72(2006.01)i； A61K9/14(2006.01)i； A61P11/00(2006.01)i

According to International Patent Classification (IPC) or to both national classification and IPC

**B. FIELDS SEARCHED**

Minimum documentation searched (classification system followed by classification symbols)

IPC:A61K,A61P

Documentation searched other than minimum documentation to the extent that such documents are included in the fields searched

Electronic data base consulted during the international search (name of data base and, where practicable, search terms used)

CNTXT; ENTXT; CNABS; DWPI; SIPOABS; CNKI; Web of Science; CNKI; 万方, WANFANG; STNext: 长风药业股份有限公司, 上海交通大学医学院附属瑞金医院, 瞿介明, 赵婧雅, 徐琳, 车琳, 李秀娟, 权梦雪, 梁文青, 表没食子儿茶素没食子酸酯, EGCG, 吸入, 气雾, 粉雾, 喷雾, 气溶胶, 肺 s 纤维化, 肺 s 间质, 弥漫性实质性肺疾病, 弥漫性实质性肺病, 弥漫性实质性肺炎, 肺间质, 结节, 石棉肺, 矽肺, 过敏性肺炎, Epigallocatechin gallate?, Epigallocatechin?, Epigallocatechin 3-gallate, Epigallocatechin 3-O-gallate?, Epigallocatechol gallate?, epi-Gallocatechin?, Pulmonary Fibrosis, PF, Interstitial Lung Disease, ILD, pneumonia, pneumonitis, Diffuse Parenchymal Lung Disease, DPLD, idiopathic interstitial pneumonia, IIPs, idiopathic, pulmonary, fibrosis, IPF, silicosis, asbestos lung?, asbestosis?, spay, inhal+, 989-51-5, 863-65-0, 435276-52-1

**C. DOCUMENTS CONSIDERED TO BE RELEVANT**

| Category* | Citation of document, with indication, where appropriate, of the relevant passages | Relevant to claim No. |
|---|---|---|
| X | US 2021401987 A1 (UNIV HOUSTON SYSTEM et al.) 30 December 2021 (2021-12-30)<br>description, paragraphs 34, 64, 254, 391-395, 450 and 416, and claims 4-6, 8-10 and 22-23 | 1-37 |
| X | CN 113384781 A (LI, Tong) 14 September 2021 (2021-09-14)<br>description, paragraph 23, and embodiment 2 | 18-22 |
| X | CN 114848707 A (BEIJING YOUYAN TECHNOLOGY CO., LTD.) 05 August 2022 (2022-08-05)<br>description, paragraphs 5 and 10-13 | 18-22 |
| X | WO 2021210033 A1 (ADAMAS BIOTECH S.R.L. et al.) 21 October 2021 (2021-10-21)<br>claims 37 and 39, and description, paragraphs 35, 41 and 48, embodiment 3, and table 4 | 1-37 |
| X | US 2011250300 A1 (THE JOHNS HOPKINS UNIVERSITY) 13 October 2011 (2011-10-13)<br>claim 40, and description, paragraphs 144 and 167, and table 1A | 1-37 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 January 2024** | **07 February 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

**INTERNATIONAL SEARCH REPORT**

International application No.
**PCT/CN2023/132299**

**C. DOCUMENTS CONSIDERED TO BE RELEVANT**

| Category* | Citation of document, with indication, where appropriate, of the relevant passages | Relevant to claim No. |
|---|---|---|
| X | US 2022000966 A1 (HOAG, G.E et al.) 06 January 2022 (2022-01-06)<br>description, paragraph 88, embodiment 10, and table 10 | 1-37 |
| X | US 2018125874 A1 (THE JOHNS HOPKINS UNIVERSITY) 10 May 2018 (2018-05-10)<br>claims 28-29, and description, paragraphs 175 and 182 | 1-37 |
| X | US 2019070145 A1 (MAYO FOUNDATION FOR MEDICAL EDUCATION AND RESEARCH) 07 March 2019 (2019-03-07)<br>description, paragraphs 23-24, 33 and 38 | 1-37 |
| X | CN 102612564 A (WUXI HEGU MEDICINE CO., LTD.) 25 July 2012 (2012-07-25)<br>claims 33, 36, 40 and 42 | 1-37 |
| X | TSAI, M.J. et al. "The Effects of Epigallocatechin Gallate (EGCG) on Pulmonary Fibroblasts of Idiopathic Pulmonary Fibrosis (IPF)—A Next-Generation Sequencing and Bioinformatic Approach"<br>*International Journal of Molecular Sciences,*<br>Vol. 20, No. (8), 22 April 2019 (2019-04-22), Document Number 1958 (pages 1-19)<br>Document Number 1958 (pages 1-19) | 1-37 |
| X | US 2022016196 A1 (UNIGEN, INC.) 20 January 2022 (2022-01-20)<br>claims 29, 33 and 49, and description, paragraphs 78-79 | 1-37 |
| X | US 2015335674 A1 (KOTTMANN, R.M. et al.) 26 November 2015 (2015-11-26)<br>claims 1, 11 and 13, and description, paragraph 139 | 1-37 |
| A | CN 101485656 A (SUZHOU CHINESE TRADITIONAL MED) 22 July 2009 (2009-07-22)<br>claim 1, and description, pages 12-13 | 1-37 |

Form PCT/ISA/210 (second sheet) (July 2022)

**INTERNATIONAL SEARCH REPORT**

International application No.
**PCT/CN2023/132299**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **23-37**
because they relate to subject matter not required to be searched by this Authority, namely:

Claims 23-37 relate to a method for preventing and/or treating pulmonary fibrosis diseases, which falls within methods for treatment of diseases as defined in PCT Rule 39.1(iv). However, a search is still performed on the basis of a pharmaceutical use thereof, i.e., the use of a (-)-epigallocatechin gallate compound in the preparation of an inhalation drug for preventing and/or treating pulmonary fibrosis diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/132299**

| Patent document cited in search report | Publication date (day/month/year) | Patent family member(s) | Publication date (day/month/year) |
|---|---|---|---|
| US 2021401987 A1 | 30 December 2021 | EP 4121111 A1 | 25 January 2023 |
| | | WO 2021188849 A1 | 23 September 2021 |
| CN 113384781 A | 14 September 2021 | None | |
| CN 114848707 A | 05 August 2022 | None | |
| WO 2021210033 A1 | 21 October 2021 | None | |
| US 2011250300 A1 | 13 October 2011 | AU 2006265113 A1 | 11 January 2007 |
| | | WO 2007005879 A2 | 11 January 2007 |
| | | WO 2007005879 A3 | 12 June 2008 |
| | | CA 2614110 A1 | 11 January 2007 |
| | | US 2015080462 A1 | 19 March 2015 |
| | | EP 1959969 A2 | 27 August 2008 |
| US 2022000966 A1 | 06 January 2022 | AU 2019365216 A1 | 10 June 2021 |
| | | SG 11202104158 SA | 28 May 2021 |
| | | MX 2021004692 A | 23 June 2021 |
| | | CA 3117213 A1 | 30 April 2020 |
| | | ZA 202103404 B | 29 November 2023 |
| | | BR 112021007692 A2 | 10 August 2021 |
| | | KR 20210119376 A | 05 October 2021 |
| | | IL 282600 A | 30 June 2021 |
| | | EP 3869985 A2 | 01 September 2021 |
| | | EP 3869985 A4 | 27 July 2022 |
| | | JP 2022509354 A | 20 January 2022 |
| | | WO 2020086759 A2 | 30 April 2020 |
| | | WO 2020086759 A3 | 22 May 2020 |
| | | WO 2020086759 A9 | 18 June 2020 |
| US 2018125874 A1 | 10 May 2018 | US 2021275561 A1 | 09 September 2021 |
| | | US 10905706 B2 | 02 February 2021 |
| | | WO 2016112271 A1 | 14 July 2016 |
| US 2019070145 A1 | 07 March 2019 | EP 3868374 A2 | 25 August 2021 |
| | | EP 3868374 A3 | 10 November 2021 |
| | | WO 2017165497 A1 | 28 September 2017 |
| | | ES 2863700 T3 | 11 October 2021 |
| | | EP 3432879 A1 | 30 January 2019 |
| | | EP 3432879 A4 | 14 August 2019 |
| | | EP 3432879 B1 | 06 January 2021 |
| | | US 2022313654 A1 | 06 October 2022 |
| CN 102612564 A | 25 July 2012 | EA 201101488 A1 | 30 July 2012 |
| | | EA 023244 B1 | 31 May 2016 |
| | | KR 20120061081 A | 12 June 2012 |
| | | US 2012071349 A1 | 22 March 2012 |
| | | US 9360471 B2 | 07 June 2016 |
| | | ES 2685947 T3 | 15 October 2018 |
| | | AU 2010233073 A1 | 01 December 2011 |
| | | AU 2010233073 B2 | 31 July 2014 |
| | | WO 2010118419 A2 | 14 October 2010 |
| | | WO 2010118419 A3 | 17 February 2011 |
| | | WO 2010118419 A4 | 21 April 2011 |
| | | EP 2965763 A1 | 13 January 2016 |
| | | EP 2965763 A9 | 02 March 2016 |

Form PCT/ISA/210 (patent family annex) (July 2022)

**INTERNATIONAL SEARCH REPORT**
**Information on patent family members**

International application No.
**PCT/CN2023/132299**

| Patent document cited in search report | Publication date (day/month/year) | Patent family member(s) | Publication date (day/month/year) |
|---|---|---|---|
| | | EP 2965763 B1 | 06 June 2018 |
| | | EP 2965763 B8 | 15 August 2018 |
| | | EP 2416792 A2 | 15 February 2012 |
| | | EP 2416792 A4 | 24 October 2012 |
| | | US 2017027914 A1 | 02 February 2017 |
| | | US 2023338343 A9 | 26 October 2023 |
| | | BRPI 1010655 A2 | 03 September 2019 |
| | | US 2010260733 A1 | 14 October 2010 |
| | | US 8492110 B2 | 23 July 2013 |
| | | JP 2015091237 A | 14 May 2015 |
| | | JP 5931164 B2 | 08 June 2016 |
| | | KR 20130128018 A | 25 November 2013 |
| | | JP 2012523239 A | 04 October 2012 |
| | | JP 5852557 B2 | 03 February 2016 |
| US 2022016196 A1 | 20 January 2022 | None | |
| US 2015335674 A1 | 26 November 2015 | US 9750761 B2 | 05 September 2017 |
| CN 101485656 A | 22 July 2009 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

• CN 202211485124 W

### Non-patent literature cited in the description

• *CHEMICAL ABSTRACTS*, 1423719-30-5